(19) Europäisches Patentamt / European Patent Office / Office européen des brevets

(11) **EP 3 422 951 B1**

(12) **EUROPEAN PATENT SPECIFICATION**

(45) Date of publication and mention
of the grant of the patent:
**22.05.2024   Bulletin 2024/21**

(21) Application number: **17760664.7**

(22) Date of filing: **28.02.2017**

(51) International Patent Classification (IPC):
*G16H 10/60* (2018.01)      *G16H 30/20* (2018.01)
*A61B 5/00* (2006.01)      *A61B 5/11* (2006.01)
*A61B 6/00* (2024.01)      *A61B 8/00* (2006.01)
*G16H 20/30* (2018.01)      *G16H 40/63* (2018.01)
*G16H 50/50* (2018.01)      *A61B 5/103* (2006.01)
*A61B 34/20* (2016.01)      *A61B 34/10* (2016.01)

(52) Cooperative Patent Classification (CPC):
**A61B 5/0004; A61B 5/1114; A61B 5/1121;
A61B 5/1122; A61B 5/1127; A61B 5/4528;
A61B 5/4533; A61B 5/4538; A61B 5/4585;
A61B 5/6828; A61B 6/461; A61B 8/461;
G16H 10/60; G16H 20/30; G16H 30/20;**      (Cont.)

(86) International application number:
**PCT/US2017/020049**

(87) International publication number:
**WO 2017/151683 (08.09.2017 Gazette 2017/36)**

(54) **CONNECTED HEALTHCARE ENVIRONMENT**

VERBUNDENE GESUNDHEITSFÜRSORGEUMGEBUNG

ENVIRONNEMENT DE SOINS DE SANTÉ CONNECTÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **29.02.2016   US 201662301417 P**

(43) Date of publication of application:
**09.01.2019   Bulletin 2019/02**

(73) Proprietor: **Mahfouz, Mohamed R.
Knoxville, TN 37922 (US)**

(72) Inventor: **Mahfouz, Mohamed R.
Knoxville, TN 37922 (US)**

(74) Representative: **Lawrie IP Limited
10 Bothwell Street
Glasgow G2 6LU (GB)**

(56) References cited:
WO-A1-2015/054290      WO-A1-2015/089118
WO-A2-2016/007936      US-A1- 2010 198 067
US-A1- 2013 217 998      US-A1- 2014 275 852

(52) Cooperative Patent Classification (CPC): (Cont.)
**G16H 40/63; G16H 50/50;** A61B 5/002;
A61B 5/1036; A61B 5/112; A61B 5/7267;
A61B 34/20; A61B 2034/105; A61B 2562/02;
A61B 2562/0219

**Description**

INTRODUCTION TO THE INVENTION

The present disclosure is directed to a connected health environment that may make use of inertial systems and related software applications to gather one or more of pre-operative, intraoperative, and post-operative data and communicate this data to a central database accessible by a clinician and patient.

WO2016007936 discloses (Abstract): "A surgical navigation module comprising: (a) a microcomputer; (b) a tri-axial accelerometer; (c) a tri-axial gyroscope; (d) at least three tri-axial magnetometers; (e) a communication module; (f) an ultrawide band transceiver; and, (g) at least four ultrawide band antennas."

WO2015089118 discloses (Abstract): "A surgical navigation system comprising a signal receiver communicatively coupled to a primary processor, the primary processor programmed to utilize a sequential Monte Carlo algorithm to calculate changes in three dimensional position of an inertial measurement unit mounted to a surgical tool, the processor communicatively coupled to a first memory storing tool data unique to each of a plurality of surgical tools, and a second memory storing a model data sufficient to construct a three dimensional model of an anatomical feature, the primary processor communicatively coupled to a display providing visual feedback regarding the three dimensional position of the surgical tool with respect to the anatomical feature."

WO2015054290 discloses (Abstract): "The present invention relates to a system and method for a digital data collection software in which a handheld electronic device collects and integrates one or more forms of data for outcome reporting. The various modules within the software can provide a patient-friendly and/or physician-friendly user interface for collecting various forms of data. The data collection templates, standardized reports and surveys can comprise consecutive predefined or custom questions or checklists, where the answers may be entered by using touch screen features, nested in menus or submenus, 3D virtual anatomical models, audio and/or visual cues. A permanent record can be generated from the collected data, where the permanent record can be synchronized for later manipulation such as optimization of the data collection template, production of reports or data analysis. All permanent records can desirably have redundant storage and compliant encryption methods."

The present invention is set out in the appended claims. Any embodiments, aspects or examples of the present description/disclosure that do not fall within the scope of the claims are provided for illustrative purposes only and do not form part of the present invention.

It is a first aspect of the present invention to provide connected healthcare environment according to claim 1 and comprising, amongst others:

(a) an electronic central data storage (central repository) communicatively coupled to at least one database comprising at least one of a statistical anatomical atlas and a kinematic database; (b) a computer running software configured to generate instructions for displaying an anatomical model of a patient's anatomy on a visual display; (c) a motion tracking device communicatively coupled to the computer and configured to transmit motion tracking data of a patient's anatomy as the anatomy is repositioned, where the software is configured to process the motion tracking data and generate instructions for displaying the anatomical model in a position that mimics the position of the patient anatomy in real time.

According to the first aspect of the invention, the at least one database comprises a statistical anatomical atlas. In a more detailed embodiment, the statistical anatomical atlas includes mathematical descriptions of at least one of bone, soft tissue, and connective tissue. In a further detailed embodiment, the mathematical descriptions are of bone, and the mathematical descriptions describe bones of an anatomical joint. In still a further detailed embodiment, the mathematical descriptions are of bone, and the mathematical descriptions describe at least one of normal and abnormal bones. In a more detailed embodiment, the mathematical descriptions may be utilized to construct a virtual model of an anatomical feature. According to the invention, the at least one database comprises a kinematic database. In another more detailed embodiment, the kinematic database includes motion data associated with at least one of normal and abnormal kinematics. In yet another more detailed embodiment, the kinematic database includes motion data associated with abnormal kinematics, and the motion data associated with abnormal kinematics includes a diagnosis for the abnormal kinematics. In still another more detailed embodiment, the motion tracking device includes an inertial measurement unit.

In yet another more detailed embodiment of the first aspect, the motion tracking device includes a plurality of inertial measurement unit. In yet another more detailed embodiment, the motion tracking device includes ultrawide band electronics. In a further detailed embodiment, the electronic central data storage is communicatively coupled to the computer. In still a further detailed embodiment, the electronic central data storage is configured to receive motion tracking data from the computer. In a more detailed embodiment, the computer is configured to send motion tracking data to the electronic central data storage. According to the invention, the central repository stores patient medical records. In another more detailed embodiment, the environment further includes a data acquisition station remote from, but communicatively coupled to, the electronic central data storage, the data acquisition station configured to access the stored patient medical records. In yet another more detailed embodiment, the stored patient medical records include a virtual anatomical model of a portion of the patient. In still another more detailed embodiment, the virtual anatomical model is a dynamic model that reflects patient movement with respect to time. In a more detailed embodiment of the first aspect, the environment further includes a machine learning data structure communicatively coupled to the electronic central data storage, the machine learning data structure configured to generate a diagnosis using the motion tracking data.

It is a second aspect of the disclosure to provide a healthcare system comprising: (a) a computer running software configured to generate instructions for displaying an anatomical model of a patient's anatomy on a visual display; (b) a motion tracking device communicatively coupled to the computer and configured to transmit motion tracking data of a patient's anatomy as the anatomy is repositioned, where the software is configured to process the motion tracking data and generate instructions for displaying the anatomical model in a position that mimics the position of the patient anatomy in real time, and where the motion tracking device includes a display.

In a more detailed embodiment of the second aspect, the computer is communicatively coupled to a statistical anatomical atlas. In yet another more detailed embodiment, the statistical anatomical atlas includes mathematical descriptions of at least one of bone, soft tissue, and connective tissue. In a further detailed embodiment, the mathematical descriptions are of bone, and the mathematical descriptions describe bones of an anatomical joint. In still a further detailed embodiment, the mathematical descriptions are of bone, and the mathematical descriptions describe at least one of normal and abnormal bones. In a more detailed embodiment, the mathematical descriptions may be utilized to construct a virtual model of an anatomical feature. In a more detailed embodiment, the computer is communicatively coupled to a kinematic database. In another more detailed embodiment, the kinematic database includes motion data associated with at least one of normal and abnormal kinematics. In yet another more detailed embodiment, the kinematic database includes motion data associated with abnormal kinematics, and the motion data associated with abnormal kinematics includes a diagnosis for the abnormal kinematics. In still another more detailed embodiment, the motion tracking device includes an inertial measurement unit.

In yet another more detailed embodiment of the second aspect, the motion tracking device includes a plurality of inertial measurement unit. In yet another more detailed embodiment, the motion tracking device includes ultrawide band electronics. In a further detailed embodiment, the system further includes an electronic central data storage communicatively coupled to the computer. In still a further detailed embodiment, the electronic central data storage is configured to receive motion tracking data from the computer. In a more detailed embodiment, the computer is configured to send motion tracking data to the electronic central data storage. In a more detailed embodiment, the electronic central data storage stores patient medical records. In another more detailed embodiment, the computer stores patient medical records that include a virtual anatomical model of a portion of the patient. In yet another more detailed embodiment, the virtual anatomical model is a dynamic model that reflects patient movement with respect to time. In still another more detailed embodiment, the system further includes a machine learning data structure communicatively coupled to the computer, the machine learning data structure configured to generate a diagnosis using the motion tracking data.

It is a third aspect not according to the invention to provide a method of acquiring medical data comprising: (a) mounting a motion tracking device to an anatomical feature of a patient, the motion tracking device including an inertial measurement unit; (b) tracking the anatomical feature with respect to time to generate position data and orientation data reflective of any movement of the anatomical feature; (c) visually displaying a virtual anatomical model of the anatomical feature, where the virtual anatomical model is dynamic and updated in real-time based upon the position data and orientation data to correspond to the positon and orientation of the anatomical feature; (d) recording changes in the virtual anatomical model over a given period of time; and, (e) generating a file embodying the virtual anatomical model and associated changes over the given period of time.

The methods disclosed, herein, do not form part of the invention which is only defined by the appended claims.

BRIEF DESCRIPTION OF THE DRAWINGS

FIG. 1 is a schematic diagram of an exemplary connected healthcare environment in accordance with the instant disclosure.

FIG. 2 is a schematic diagram of an exemplary connected health workflow between a patient and physician/clinician in accordance with the foregoing environment of FIG. 1

FIG. 3 is a screen shot from an exemplary data acquisition device showing a pair of exemplary pods available to be paired with the data acquisition device.

FIG. 4 is a screen shot from an exemplary data acquisition device showing an address of a pod already having been registered to the exemplary data acquisition device.

FIG. 5 is a screen shot from an exemplary data acquisition device showing a starting step for initiating calibration sequence for an exemplary pod.

FIG. 6 is a screen shot from an exemplary data acquisition device providing instructions to a user on how to rotate the pod in order to perform a first step of an exemplary calibration sequence.

FIG. 7 is a screen shot from an exemplary data acquisition device providing instructions to a user on how to rotate the pod in order to perform a second step of an exemplary calibration sequence.

FIG. 8 is a screen shot from an exemplary data acquisition device providing instructions to a user on how to rotate the pod in order to perform a third step of an exemplary calibration sequence.

FIG. 9 is a screen shot from an exemplary data acquisition device after completion of the third step of an exemplary calibration sequence.

FIG. 10 shows local magnetic field maps (isometric, front, and top views) generated from data output from and IMU before calibration (top) and data from the IMU post calibration (bottom) where the plots resemble a sphere.

FIG. 11 is a series of diagrams showing exemplary locations of magnetometers associated with an IMU, what the detected magnetic field from the magnetometers should be to reflect post normalization to account for magnetic distortions.

FIG. 12 is an exemplary process flow diagram for soft tissue and kinematic tracking of body anatomy using IMUs in accordance with the instant disclosure.

FIG. 13 is a screen shot from an exemplary data acquisition device showing pods having been previously registered with the data acquisition device and ready for use in accordance with the instant disclosure.

FIG. 14 is a screen shot from an exemplary data acquisition device showing a plurality of exercise motions that may be selected for greater precision of kinematic tracking.

FIG. 15 is a screen shot from an exemplary data acquisition device showing a user of the pods the proper placement of the pods on the patient for data acquisition.

FIG. 16 is a picture showing a patient having pods mounted to lower and upper legs consistent with the indications shown in FIG. 15.

FIG. 17 is a screen shot from an exemplary data acquisition device showing a confirmation button a user must press to initiate motion tracking in accordance with the instant disclosure.

FIG. 18 is a screen shot from an exemplary data acquisition device showing a virtual anatomical model of a patient's knee joint prior to data acquisition.

FIG. 19 is a screen shot from an exemplary data acquisition device showing a virtual anatomical model of a patient's knee joint 19 seconds into data acquisition.

FIG. 20 is a screen shot from an exemplary data acquisition device showing a virtual anatomical model of a patient's shoulder joint prior to data acquisition.

FIG. 21 is a screen shot from an exemplary data acquisition device showing a saved file of a dynamic virtual anatomical model over a range of motion that is available for playback on the data acquisition device.

FIG. 22 is a photograph of the rear, lower back of a patient showing separate inertial measurement units (IMU) placed over the L1 and L5 vertebrae for tracking relative motion of each vertebra through a range of motion, as well as an ancillary diagram showing that each IMU is able to output data indicative of motion across three axes.

FIG. 23 comprises a series of photographs showing the patient and IMUs of FIG. 175 while the patient is moving through a range of motion.

FIG. 24 is a graphical depiction representative of a process for determining the relative orientation of at least two bodies using inertial measurement unit data in accordance with the instant disclosure.

FIG. 25 is an exemplary illustration of a clinical examination of a knee joint using inertial measurement units to record motion data in accordance with the instant disclosure.

FIG. 26 is an exemplary illustration of a clinical examination of a knee joint using inertial measurement units to record motion data in accordance with the instant disclosure.

FIG. 27 is an exemplary illustration of a clinical examination of a knee joint using inertial measurement units to record motion data in accordance with the instant disclosure.

FIG. 28 is an exemplary illustration of a clinical examination of a knee joint using inertial measurement units to record motion data in accordance with the instant disclosure.

FIG. 29 is an exemplary illustration of a clinical examination of a knee joint using inertial measurement units to record motion data in accordance with the instant disclosure.

FIG. 30 is an exemplary illustration of a clinical examination of a knee joint using inertial measurement units to record motion data in accordance with the instant disclosure.

FIG. 31 is an exemplary illustration of a clinical examination of a knee joint using inertial measurement units to record motion data in accordance with the instant disclosure.

FIG. 32 is an exemplary illustration of a clinical examination of a knee joint using inertial measurement units to record motion data in accordance with the instant disclosure.

FIG. 33 is an exemplary illustration of a clinical examination of a knee joint using inertial measurement units to record motion data in accordance with the instant disclosure.

FIG. 34 is a profile and overhead view of an exemplary UWB and IMU hybrid tracking system as part of a tetrahedron module.

FIG. 35 is an illustration of an exemplary central and peripheral system in a hip surgical navigation system. The image on the left shows one of the anchor interrogating the peripheral unit's tags at one instance of time, and the image on the right shows a different anchor interrogating the peripheral unit's tags at the following instance of time. Each anchors interrogate the tags in the peripheral unit to determine the translations and orientations relative to the anchors.

FIG. 36 is a diagram of an experimental setup of UWB antennas in an anechoic chamber used to measure the UWB antenna 3-D phase center variation. A lookup table of phase center biases is tabulated during this process and used to mitigate phase center variation during system operation.

FIG. 37 is an exemplary block diagram of the hybrid system creating multiple tags with a single UWB transceiver.

FIG. 38 is an exemplary block diagram of UWB transmitter in accordance with the instant disclosure.

FIG. 39 is an exemplary diagram showing how to calculate the position of a tag based upon TDOA.

FIG. 40 is an exemplary block diagram for the processing and fusion algorithm of the UWB and IMU systems.

FIG. 41 is an overhead view of a central unit and peripheral unit in an experimental setup. The central unit remains stationary while the peripheral unit is maneuvered during the experiment.

FIG. 42 is an exemplary block diagram of preoperative preparation and surgical planning, and the intraoperative use of the surgical navigation system to register patient with the computer.

FIG. 43 is an illustration of using one central unit on the pelvis and a minimum of one peripheral unit to be used on the instrument.

FIG. 44 is an illustration of using one central unit adjacent to the operating area, a peripheral unit on the pelvis and a minimum of one peripheral unit to be used on the instruments.

FIG. 45 is an illustration of using one central unit and a peripheral unit to obtain cup geometry of the patient for registration.

FIG. 46 is an illustration of using one central unit and a peripheral unit to perform surgical guidance in the direction and depth of acetabular reaming.

FIG. 47 is an illustration of attachment of the peripheral unit to the acetabular shell inserter.

FIG. 48 is an illustration of attachment of the peripheral unit to the femoral broach.

DETAILED DESCRIPTION

The exemplary embodiments of the present disclosure are described and illustrated below to encompass exemplary connected health environments that may make use of inertial systems and related software applications to gather one or more of preoperative, intraoperative, and post-operative data and communicate this data to a central database accessible by a clinician and patient. Of course, it will be apparent to those of ordinary skill in the art that the embodiments discussed below are exemplary in nature and may be reconfigured within the scope of the present invention, that is solely defined by the appended claims. However, for clarity and precision, the exemplary embodiments as discussed below may include optional steps, methods, and features that one of ordinary skill should recognize as not being a requisite to fall within the scope of the present invention.

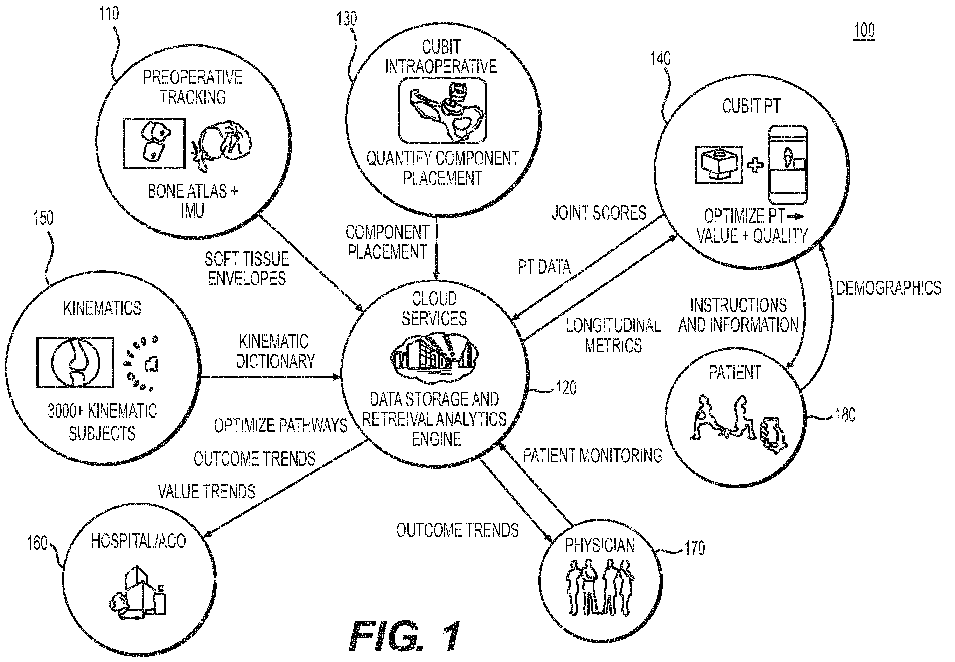

Referencing FIG. 1, an exemplary schematic diagram of a connected healthcare environment 100 that may make use numerous databases and inertial systems to gather one or more of pre-operative, intraoperative, and post-operative data, which is aggregated in a central database, either locally or in some remote storage location, that is accessible to clinicians/physicians and patients. This operative data may include quantitative data resulting from combining inertial data with more qualitative scores (such as scores for a patient's joint) and patient reported experiences to allow all stakeholders in the healthcare ecosystem to make more informed treatment decisions.

Connected healthcare and telemedicine are becoming increasingly important as pressure mounts to decrease cost and improve quality of care. Current networked solutions rely on direct patient-to-physician contact to gather qualitative information regarding patient status. While this provides value in reducing in-person visits, the data gathered usually must be transferred by a person from paper to digital format - if any data is collected at all. The instant disclosure, however, provides a connected healthcare environment 100 solution that may incorporate inertial measurement units (IMUs), consisting of accelerometers, gyroscopes, and magnetometers, into the clinical pathway to enhance qualitative and quantitative data collection and allow for direct analytical measurements and outcomes reporting. This exemplary connected healthcare environment may include the following components, a more detailed explanation of which is provided as follows.

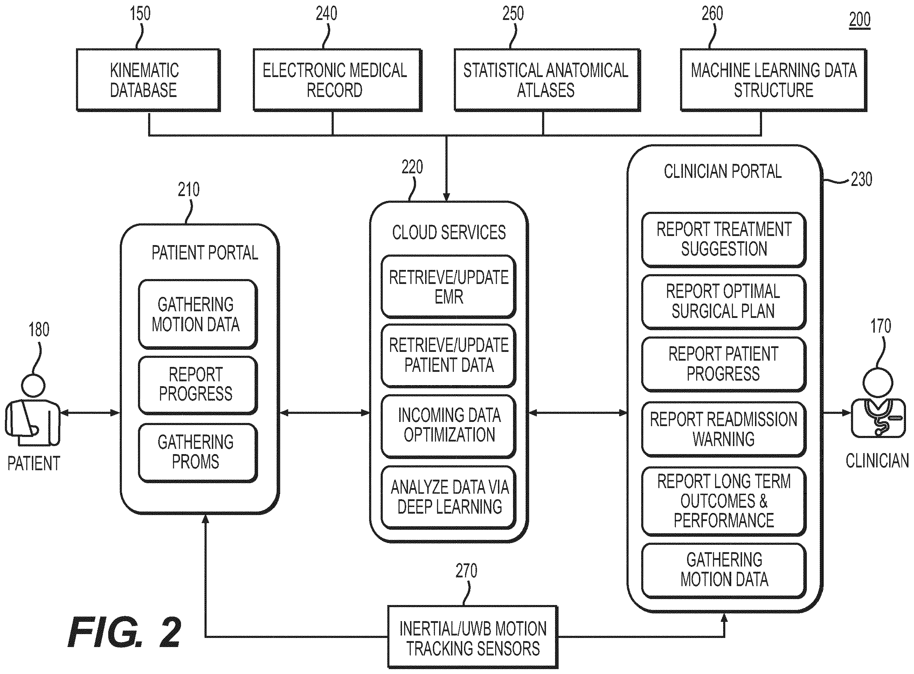

A first component of the exemplary environment 100 comprises a pre-operative tracking aspect 110. This tracking aspect 110 may include a combination of hardware and software that may be utilized to track the motion of a patient's body part(s) in load bearing and non-load bearing ranges of motion. In exemplary form, the hardware may include a kinematic monitoring device comprising IMUs and, optionally, ultra-wide band (UWB) electronics (individually or collectively, reference 270, see FIG. 2) that may be used in a pre-operative setting as a way of capturing soft tissue envelopes - important for many total joint procedures. In order to capture motion data, each monitoring device is placed in a known orientation on the patient to provide recorded motion of one or more body parts. By way of example, each monitoring device may be mounted to a patient's bone comprising a portion of a joint in order to record motion of the joint (n joints requires $n+1$ monitoring devices). In order to make the tracked motion more accurate, the tracking aspect 110 may make use of virtual anatomical models derived from anatomical image data. A more detailed discussion of the tracking devices will be made later in the instant disclosure. Nevertheless, the tracking devices provide data indicative of changes in position and orientation of the tracked anatomy across a range of motion. This tracking data/information is recorded locally on a hand-held or tabletop device and transmitted to the central repository aspect 120 of the environment 100.

Virtual anatomical three dimensional models may be associated with data output from the tracking devices in order to provide a visual feedback element representing how the patient's bones and soft tissues may be moving relative to one another across a range of motion. By way of example, pre-operative anatomical image data (CT, X-ray) may be segmented, or an imaging modality such as magnetic resonance imaging (MRI) that is naturally segmented, may be utilized to create virtual three dimensional anatomical models. Those skilled in the art are familiar with techniques utilizing segmented anatomical images and creating virtual anatomical models therefrom and, accordingly, a detailed discussion of this aspect has been omitted in furtherance of brevity. Presuming only a bone model is segmented, one may then identify the segmented bone and reference an anatomical atlas specific to that bone in order to identify soft tissue locations on the bone in question. The anatomical atlas data may be used to identify soft tissue locations and overlay a virtual model of the soft tissue structures onto the bone model using prior information on soft tissue (ligament) attachment sites. For example, the bone atlas may have information for the medial collateral ligament attachment site on a femur stored as a bone landmark so that the landmark and soft tissue can be associated with the patient-specific bone model.

The exemplary statistical atlas 250 in accordance with the instant disclosure may comprise one or more modules/databases (see FIG. 2). By way of example, the modules may comprise a tissue and landmark module, an abnormal anatomical module, and a normal anatomical module. In exemplary form, each module comprises a plurality of mathematical representations of a given population of anatomical features (bones, tissues, etc). The normal anatomical module of the atlas allows automated measurements of anatomies in the module and reconstruction of missing anatomical features. The module can be specific to an anatomy or contain a plurality of anatomies. For bone, a useful input and output are three-dimensional surface models. The bone anatomical module can be used to first derive one or both of the following outputs: (1) a patient specific anatomical construction (output is patient specific anatomical model), (2) a template which is closest to the patient specific anatomy as measured by some metric (surface-to-surface error is most common). If an input anatomical model (not belonging to the module) is incomplete, as in the case of the abnormal database, then a full bone reconstruction step can be performed to extract appropriate information. A second module, the abnormal anatomical module, includes mathematical representations of a given population of anatomical features consisting of anatomical surface representations and related clinical and ancestral data. Data from this second module may be used an input to generate a reconstructed full anatomical virtual model representative of normal anatomy. A third module, the soft tissue and landmark module, comprises mathematical representations of feature or regions of interest on anatomical models. By way of example, the features or regions of interest may be stored as a set of numbered vertices, with numbering corresponding to the virtual anatomical model. Using the knee as an example, the medial collateral ligament can be represented in this module as a set of vertices belonging to the attachment site based on a series of observed data (from cadavers or imaging sets). In this fashion, the vertices from this module may be propagated across a population of the normal module to identify a corresponding region on each model in the population. And these same vertices may be associated with a patient-specific anatomical model to identify where on the anatomical model the corresponding region would be located.

These patient-specific anatomical models (with or without soft tissue) may be utilized by the tracking aspect 110 to associate the tracked position data, thereby providing visual feedback and quantitative feedback concerning the position of certain patient anatomy and how this position changes with respect to other patient anatomy across a range of motion. By teaming a patient-specific anatomical model with kinematic motion tracking, the pre-operative aspect 110 provides dynamic data representative of the anatomical model changing positions consistent with the tracked motion. For example, in the context of a knee joint, the anatomical model may comprise a patient's femur and tibia, where the kinematic motion tracking data is associated with the models of the femur and tibia to create a dynamic model of the patient's femur and tibia that move with respect to one another across the range of motion tracked using the monitoring devices. And this dynamic model may be utilized by a clinician to diagnose a degenerative or otherwise abnormal condition and suggest a corrective solution that may include, without limitation, partial or total anatomical reconstruction (such as joint reconstruction using a joint implant) and surgical pre-planning to ensure that bone resections do not violate the patient specific soft tissue envelope.

In summary, the tracking aspect 110 includes a series of data inputs that may include various sources. By way of example, the sources may include, without limitation, motion data based upon exercise or physician manipulation, medical history data from medical records and demographics, strength data, anatomical image data, and qualitative and quantitative data concerning joint condition and pain levels experienced by the patient. These data inputs may be forwarded to a machine learning data structure 260 (see FIG. 2) for a diagnostic analysis, as well as to a statistical atlas for measurement of various anatomical features.

In addition to data inputs, the tracking aspect 110 may send out various data to communicatively coupled aspects. By way of example, the output data may include, without limitation, surgical planning data, suggested surgical technique data, preferred intervention methods, and anatomical measurements from an anatomical atlas that may include joint spacing measurements and location of kinematic axes.

A second component of the exemplary environment 100 comprises an intraoperative surgical navigation aspect 130. The navigation aspect 130 may include two or more IMUs 270 for tracking anatomical position and orientation during surgery. Moreover, the navigation aspect 130 may include two or more IMUs for tracking surgical tool/instrument position and orientation during surgery. Further, the navigation system 130 may include two or more IMUs 270 for tracking orthopedic implant position and orientation during surgery. The foregoing tracking can be performed in an absolute sense or relative to a surgical plan created in software prior to operating. While the navigation aspect may utilize two or more IMUs, it is also within the scope of this disclosure to integrate the IMUs with UWB electronics 270 to create additional position information that may be utilized as a check or to further refine the position data generated by the IMUs. In this fashion, one can use IMUs and UWB electronics 270 to track both position and orientation of orthopedic implant components, anatomical structures, and surgical instruments/tools. A more detailed discussion of how the IMUs and UWB electronics 270 are integrated is provided later in this disclosure. Nevertheless, position and orientation data from the IMUs and UWB electronics 270 is sent wirelessly to a processing device in the operating room, where the data is recorded. The recorded position and orientation data for a patient case/surgery is sent, through a computer network, to the central repository 120 where the data is associated with the patient's electronic records.

A third component of the exemplary environment 100, according to the invention, comprises a post-operative physical therapy (PT) aspect 140. The PT aspect 140 may comprise IMUs and, optionally, UWB electronics 270 as part of monitoring devices used to monitor patient rehabilitation exercises. The monitoring devices are placed in a known orientation on the patient (similar to the process discussed above for the pre-operative tracking aspect 110, including loading of patient-specific virtual anatomical models) in order to generate and record data indicative of the motion of the patient's anatomy (such as a joint, where each joint (*n*) may require *n+1* monitoring devices). During a specified activity, the anatomy motion is tracked in real time by the tracking device 270 (IMU or IMU+UWB) and sent wirelessly to a hand-held or tabletop device such as, without limitation, a smart telephone, a laptop computer, a desktop computer, and a tablet computer. By way of example, the hand-held device may be the patient's smart telephone and this telephone may relay additional information to the patient regarding appropriate movements during the rehabilitation exercise to ensure addressing the correct range of motion and form, as well as counseling against exceeding maximum ranges of motion for certain excercises. In exemplary form, the hand-held device can display a dynamic anatomical model that duplicates the patient's actual motion and the hand-held device can record video of this dynamic model movement. All or portions of the collected data may be sent through a network to the central repository 120 where the data is associated with the patient's electronic records. The updated patient records may then be accessible by a physician or therapist to confirm rehabilitation technique and frequency, get progress reports over time, and bill for telemedicine services. Likewise, according to the invention, patients can access their own medical records in the central repository 120 and obtain information for status on recovery goals and metrics.

In summary, data inputs to the PT aspect 140 may include a series of data inputs that may include various sources. By way of example, the sources may include, without limitation, motion data based upon exercise or physician manipulation, medical history data from medical records and demographics, strength data, anatomical image data, and qualitative and quantitative data concerning joint condition and pain levels experienced by the patient. These data inputs may be forwarded to a machine learning data structure 260 (see FIG. 2) for a diagnostic analysis, as well as to a statistical atlas for measurement of various anatomical features.

In addition to data inputs, the PT aspect 140 may include a series of data outputs. In exemplary form, the data outputs may comprise, without limitation, reported patient outcomes, physical therapy metrics, and warning indicators indicative of readmission to perform surgical intervention.

A main hub of the exemplary environment 100 is the central repository 120. In exemplary form, the central repository 120 comprises a local or cloud based storage of patient information and data. The central repository 120 provides access and reports customized for all stakeholders (patients, hospitals, physicians, etc.) of the environment 100 via an access portal. In exemplary form, the access portal may comprise a mobile application on a smart telephone, software running on a tablet, laptop, or desktop computer or server.

A fourth component of the exemplary environment 100 comprises a kinematic database aspect 140. The kinematic database aspect 140 may comprise a kinematic dictionary containing kinematic profiles of multiple normal, abnormal, and implanted subjects, as well as a determination whether the kinematic profile was collected preoperatively, post-operatively, and intraoperatively. In exemplary form, the kinematic database aspect 140 may comprise a plurality of kinematic datasets (motion) and respective measurements extracted from each dataset. Measurements may include axes, spacing, contact, soft tissue lengths, time of exercise as well as subject demographics. Newly acquired kinematic data may be measured against this database aspect 140 to create predictions on pathological severity (if patient is using pre-operative data capture), optimal treatment pathways or functional rehabilitation objectives. The kinematic database aspect 140 may also be used to create appropriate training and testing data to be used as input into deep learning networks 260 (see FIG. 2) or similar machine learning algorithms to provide appropriate input/output relationships. In this fashion, the kinematic database aspect 140 includes kinematic data that is aggregated and analyzed for correlations, trends, and bottle necks. For example, the orientation data from the PT aspect 140 may be combined with position data from the kinematic database aspect 140 to estimate full position and orientation tracking in circumstances where UWB electronics may not be utilized. Moreover, the kinematic database aspect 140 includes identifiers associated with the data that corresponds to particular diagnoses so that by comparing data from the pre-operative tracking aspect 110 may allow a physician to diagnose a patient with a particular diagnosis or to confirm a diagnosis by showing how analytic data corresponds well to other like diagnoses.

It should be noted that while the a kinematic database aspect 140 has been described as a kinematic database communicatively coupled to the central repository 120, it is also within the scope of the disclosure to communicatively couple additional resources and databased such as statistical anatomical atlases as described in more detail hereafter. Moreover, it is also within the scope of the disclosure to communicatively couple machine learning (deep learning) structures to the central repository 120. Examples of this can be seen in FIG. 2 and will be discussed in more detail hereafter and beforehand.

While not dedicated aspect per se, the exemplary environment includes access portals 160, 170 for hospitals and physicians in order to access the data generated by the aspects 110, 130, 140 and incorporated into the patient records at the central repository 120. At the same time, the central repository 120 may act as a conduit through which the aspects 110, 130, 140 gain access to information in the kinematic database aspect 150, as well as hospitals and physicians gaining access to the kinematic database aspect. In general, hospitals may utilize the central repository 120 to optimize pathways to successful treatment, observe trends associated with patient outcomes, and evaluate patient outcome trends to quantitatively and qualitatively assess various treatment and rehabilitation options. Likewise, physicians may utilize the central repository 120 to optimize pathways to successful treatment, observe trends associated with patient outcomes, and evaluate patient outcome trends to quantitatively and qualitatively assess various treatment and rehabilitation options, monitor patients, and diagnose patient conditions. Though the central repository 120 is not depicted as being directly linked to the patient 180, given that at least some of the patient information is immediately accessible to the patient using certain aspects 110, 140, it should be known that the central repository 120 may provide a link that allows patients to review only their own patient data and associated metrics concerning any post-operative treatment or rehabilitation.

Referring to FIG. 2, a schematic diagram illustrates an exemplary connected health workflow 200 between a patient and physician/clinician in accordance with the foregoing environment 100 (see FIG. 1). In particular, a patient portal 210 comprises a software interface for collecting data from the foregoing monitoring devices (IMUs, UWB electronics, etc.), interfacing with the central repository 120 (specifically, the cloud services 220), and reporting data to the patient 180. The patient portal 210 is designed to allow the patient access as needed, and can be deployed on any computing device including, without limitation, a laptop or mobile device for portability. The patient portal 210 serves as the software interface that gathers kinematic and motion data associated with the PT and tracking aspects 140, 110, reports progress such as range of motion performance during physical therapy, and gathers patient reported outcome measures (PROMS).

As discussed previously, gathering kinematic and motion data via either the tracking or PT aspects 110, 140 of the environment 100 may be performed using motion tracking devices (IMUs, UWB electronics, etc.) that are attached to the anatomy or anatomical region of interest of the patient 180. The tracking devices communicate wirelessly with patient portal 210 in order to transmit sensor data reflecting changes in orientation and position of the anatomy or anatomical region of interest. According to the invention, the patient portal 210 is operative to utilize the sensor data to determine changes in orientation and position of the anatomy or anatomical region of interest, as well as generating instructions for dynamically displaying a virtual anatomical model being dynamically repositioned in real time to mimic the motion of the patient's anatomy. In exemplary form, to the extent the patient portal 210 is associated with a smart telephone, the dynamic model may be displayed and updated in real-time on the visual screen of the telephone. Likewise, the patient portal 210 may be in communication with a memory associated with the telephone or remote from the telephone to allow the memory to store the dynamic data generated from the sensors. In this fashion, the dynamic data may be accessed and utilized to generate a stored version of the dynamic anatomical virtual model.

According to the invention, post orientation and position data collection, the patient portal 210 utilizes a wired or wireless interface with the cloud services 220 (part of the central repository 120) to analyze the data, create reports for the patient that provide indicators of recovery progress, and update the patient's electronic medical records with the new information. Reporting progress allows the patient 180 to update their own quantitative performance metric such as, without limitation, a range of motion during physical therapy. The accumulated data by the patient portal 210 provide precise and objective assessment that may be used by a physician 170 to prescribe the optimal exercises based on the patient's current or past performance.

Integrated within the patient portal 210 may be a series of standard questions related to PROMS. The patient portal 210 may regularly query the patient 180 to answer questions related to satisfaction and functional scores. Standard questionnaires can be utilized here, such as Oxford Knee Score, Oxford Hip Score, EQ-5D, or other methodologies. When collected, this data can be uploaded to the cloud services 220 (part of the central repository 120) for integration into the patient record and utilized to assess progress through a clinician portal 230.

Referring again to FIGS. 1 and 2, as used herein, cloud services 220 is intended to refer to any of the growing number of distributed computing services accessible through network infrastructure. This includes remote databases, machine learning calculations, remote electronic medical records, and any other form of internet enabled data management and computational support. In this exemplary environment 100, the cloud services 220 may be utilized to access and update information related to kinematic data, such as the kinematic database aspect 150, statistical anatomical databases, machine learning structures 260 (training sets, test sets and/or previously trained deep learning networks), as well as an interface for communicating with existing electronic medical record infrastructures. The cloud services 220 may handle communication to and from the patient and clinician portals 210, 230 to facilitate transfer of appropriate data when queried. This includes retrieving\updating the patient electronic medical records (EMR) 240 with data when it is collected or when patient information is updated. This also includes retrieving\updating patient data that may not be stored in the EMR, but may be accessed as part of the environment 100, such as inertial data (position, orientation), kinematic data (whether raw or processed), and patient specific anatomical virtual models.

A significant function of the cloud services 220 in the exemplary connected healthcare environment 100 is to collect and organize incoming motion and patient data at the point of care (through the patient 210 or clinical portal 230) and distribute that data to aspects responsible for analysis. One form of analysis is taking input motion data and associated anatomical measurements collected as part of the pre-operative tracking aspect 110 and outputting a diagnosis and appropriate or optimal treatment strategy. If arthroplasty is a treatment option, the analysis may also output optimal surgical planning results, such as implant sizing and implant placement, based on the kinematic data and anatomical models. This plan may be tailored to optimize ligament balance, restore a joint line, or reduce implant loading for potentially longer lasting implants. In a post-operative or rehabilitation setting, as part of the PT aspect 140, the data may be analyzed to optimize patient exercise routines or warn of potential issues or setback that may lead to readmission, thereby allowing preventative adjustment to treatment. Independent of the setting, converting motion data to useful information may require sophisticated machine learning techniques that include, without limitation, deep learning. Deep learning involves mapping a series of inputs to specific outputs after a training and testing optimization period. A deep learning network 260 can be fed new data as input and utilize learned weighting to determine the associated output. For connected health environment 100, the input information can take the form of kinematic data and patient information and output could be, among other things, likely diagnosis, appropriate treatment, or a score related to functional performance. The computational aspect of mapping new inputs into outputs is offloaded from the point of care software applications and performed on the cloud services 220, which distributes the computational effort and reports back to the application(s) from which the data was generated as well as to the clinician portal 230.

The clinician portal 230 comprises a software interface for interfacing with the appropriate cloud services 220 and reporting data to the clinician 170 in the connected healthcare workflow environment 100. The clinician portal 230 is designed to allow the clinician 170 to be connected and accessed relevant information for their patients, and can be deployed on any computing device such as, without limitation, a desktop computer, a laptop computer, a tablet, or any other processor-based device. The clinician 170 can use the portal 230 to gather new motion data (with inertial sensors), monitor patient status\progress, retrieve motion analysis results in the form of treatment suggestions, diagnostic suggestions, optimized surgical plans, readmission warnings if a patient is not progressing or is regressing.

In this exemplary environment 100, both the patient and clinician portals 210, 230 have the appropriate mechanisms for communicating with the IMUs and UWB electronics 270. Also, both patient and clinician portals 210, 230 include functionality for recording data, calibrating sensors 270 and coupling this information to alternative sensing systems.

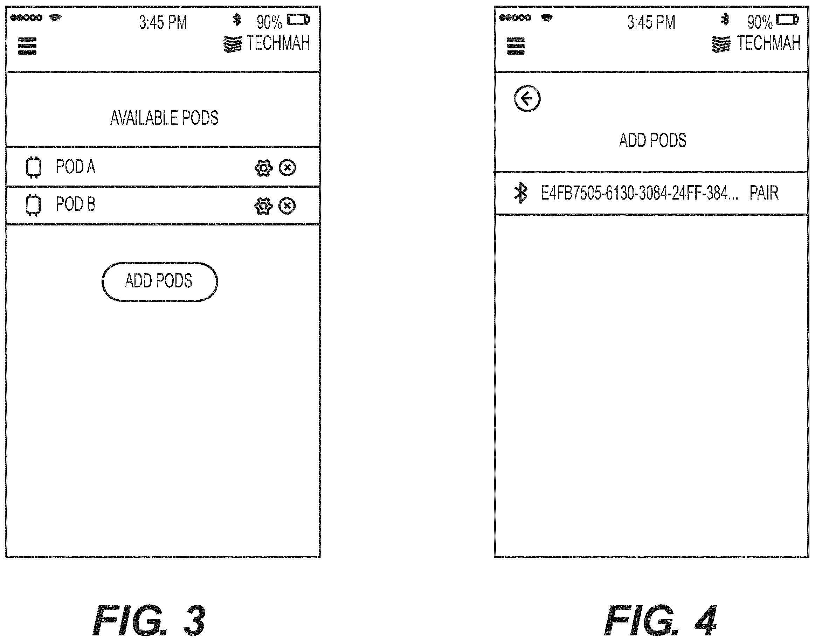

Referring to FIGS. 3 and 4, the exemplary environment 100 may make use of tracking devices 270 that include IMUs and UWB electronics. A more detailed discussion of these hardware components is included later in the instant disclosure. These tracking devices 270 or "Pods" may be mounted to an anatomy of a patient to track the kinematic motion of the anatomy. For purposes of explanation only, the anatomy will be described as a knee joint. Nevertheless, those skilled in the art will understand that other body parts of a patient may be motion tracked such as, without limitation, any bone or bones of the patient including the bones of the hip joint, the ankle joint, the shoulder joint.

In exemplary form, the exemplary environment 100 includes a plurality of Pods 270. In order to utilize the Pods, which may be wireless, each Pod 270 must be activated, which may occur via remote activation through the patient portal 210 or locally by manually switching power on to the Pod. Post powering on one or more Pods 270, a connection step may be undertaken to pair each Pod with a data reception device, such as a smart telephone. Pairing between each Pod and the data reception device may be via Bluetooth or any other communication protocol so that the patient portal 210 (running on the smart telephone or any other processor based device) receives data from the Pods. In the context of a Bluetooth connection, the connecting device (e.g., a smart telephone) may include a screen interface that identifies all available Pods 270 for pairing (see FIG. 3). A user of the smart telephone (running the patient portal 210) need only select one or more Pods the user desires to pair. FIG. 4 shows a screen shot from an exemplary smart telephone identifying at least one of the Pods has successfully been paired.

The IMUs 270 of the instant disclosure are capable of reporting orientation and translational data reflective of changes in position and orientation of the objects to which the IMUs are mounted. These IMUs 270 are communicatively coupled (wired or wireless) to a software system, such as the patient portal 210, that receives output data from the IMUs indicating relative velocity and time that allows the software of the portal 210 to calculate the IMU's current position and orientation, or the IMU 270 calculates and sends the position and orientation information directly to portal. In this exemplary description, each IMU 270 may include three gyroscopes, three accelerometers, and three Hall-effect magnetometers (set of three, tri-axial gyroscopes, accelerometers, magnetometers) that may be integrated into a single circuit board or comprised of separate boards of one or more sensors (e.g,, gyroscope, accelerometer, magnetometer) in order to output data concerning three directions perpendicular to one another (e.g., X, Y, Z directions). In this manner, each IMU 270 is operative to generate 21 voltage or numerical outputs from the three gyroscopes, three accelerometers, and three Hall-effect magnetometers. In exemplary form, each IMU 270 includes a sensor board and a processing board, with a sensor board including an integrated sensing module consisting of a three accelerometers, three gyroscopic sensors and three magnetometers (LSM9DS, ST-Microelectronics) and two integrated sensing modules consisting of three accelerometers, and three magnetometers (LSM303, ST-Microelectronics). In particular, the IMUs 270 each include angular momentum sensors measuring rotational changes in space for at least three axes: pitch (up and down), yaw (left and right) and roll (clockwise or counter-clockwise rotation). More specifically, each integrated sensing module magnetometer is positioned at a different location on the circuit board, with each magnetometer assigned to output a voltage proportional to the applied magnetic field and also sense polarity direction of a magnetic field at a point in space for each of the three directions within a three dimensional coordinate system. For example, the first magnetometer outputs voltage proportional to the applied magnetic field and polarity direction of the magnetic field in the X-direction, Y-direction, and Z-direction at a first location, while the second magnetometer outputs voltage proportional to the applied magnetic field and polarity direction of the magnetic field in the X-direction, Y-direction, and Z-direction at a second location, and the third magnetometer outputs voltage proportional to the applied magnetic field and polarity direction of the magnetic field in the X-direction, Y-direction, and Z-direction at a third location. By using these three sets of magnetometers, the heading orientation of the IMU may be determined in addition to detection of local magnetic field fluctuation. Each magnetometer uses the magnetic field as reference and determines the orientation deviation from magnetic north. But the local magnetic field can, however, be distorted by ferrous or magnetic material, commonly referred to as hard and soft iron distortion. Soft iron distortion examples are materials that have low magnetic permeability, such as carbon steel, stainless steel, etc. Hard iron distortion is caused by permanent magnets. These distortions create a non-uniform field (see FIG. 184), which affects the accuracy of the algorithm used to process the magnetometer outputs and resolve the heading orientation. Consequently, as discussed in more detail hereafter, a calibration algorithm may be utilized to calibrate the magnetometers to restore uniformity in the detected magnetic field. Each IMU 270 may be powered by a replaceable or rechargeable energy storage device such as, without limitation, a CR2032 coin cell battery and a 200mAh rechargeable Li ion battery.

The integrated sensing modules as part of the IMUs 270 may include a configurable signal conditioning circuit and analog to digital converter (ADC), which produces the numerical outputs for the sensors. The IMU 270 may use sensors with voltage outputs, where an external signal conditioning circuit, which may be an offset amplifier that is configured to condition sensor outputs to an input range of a multichannel 24 bit analog-to-digital converter (ADC) (ADS 1258, Texas Instrument). The IMU 270 may further include an integrated processing module that includes a microcontroller and a wireless transmitting module (CC2541, Texas Instrument). Alternatively, the IMU 270 may use separate low power microcontroller (MSP430F2274, Texas Instrument) as the processor and a compact wireless transmitting module (A2500R24A, Anaren) for communication. The processor may be integrated as part of each IMU 270 or separate from each IMU, but communicatively coupled thereto. This processor may be Bluetooth compatible and provide for wired or wireless communication with respect to the gyroscopes, accelerometers, and magnetometers, as well as provide for wired or wireless communication between the processor and a signal receiver.

Each IMU 270 is communicatively coupled to a signal receiver, which uses a predetermined device identification number to process the received data from multiple IMUs. The data rate is approximately 100 Hz for a single IMU and decreases as more IMUs join the shared network. The software of the signal receiver receives signals from the IMUs 270 in real-time and continually calculates the IMU's current position based upon the received IMU data. Specifically, the acceleration measurements output from the IMU are integrated with respect to time to calculate the current velocity of the IMU in each of the three axes. The calculated velocity for each axis is integrated over time to calculate the current position. But in order to obtain useful positional data, a frame of reference must be established, which may include calibrating each IMU.

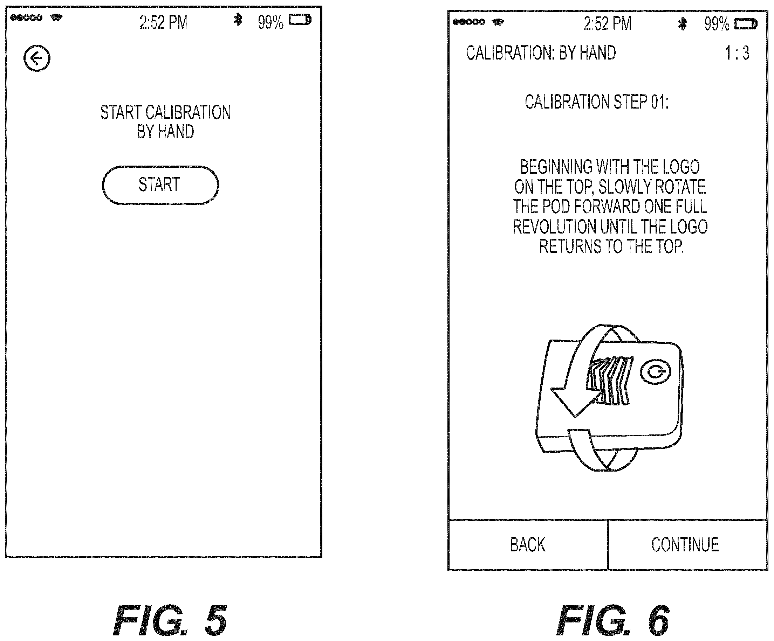

Referring to FIGS. 5-9, the goal of the calibration sequence is to establish zero with respect to the accelerometers of the Pods 270 (i.e., meaning at a stationary location, the accelerometers provide data consistent with zero acceleration) within three orthogonal planes and to map the local magnetic field and to normalize the output of the magnetometers to account for directional variance and the amount of distortion of the detected magnetic field. In order to calibrate the accelerometers of the Pods 270, multiple readings are taken from all accelerometers at a first fixed, stationary position. As shown in FIG. 5, the user of the smart telephone may actuate a calibration sequence by using the patient portal 210 to start a manual calibration sequence for each Pod 270.

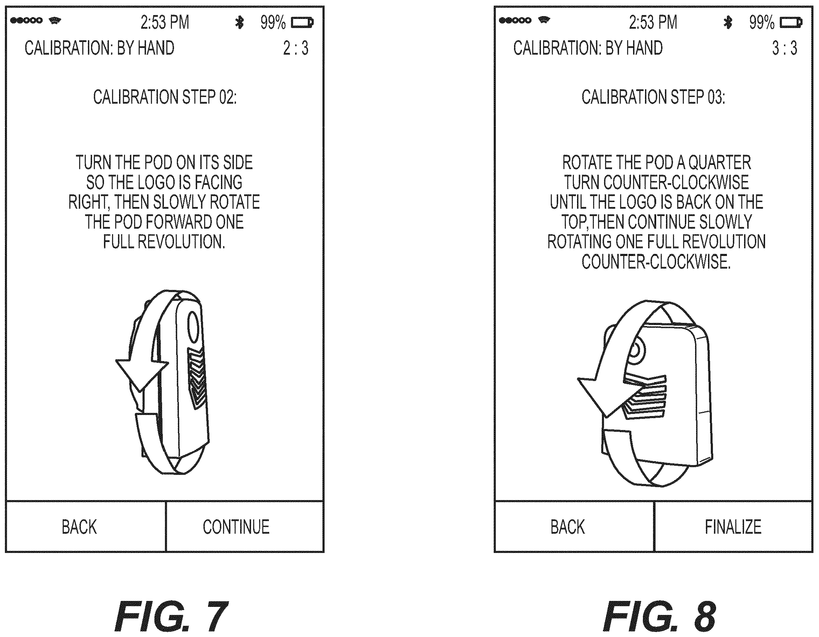

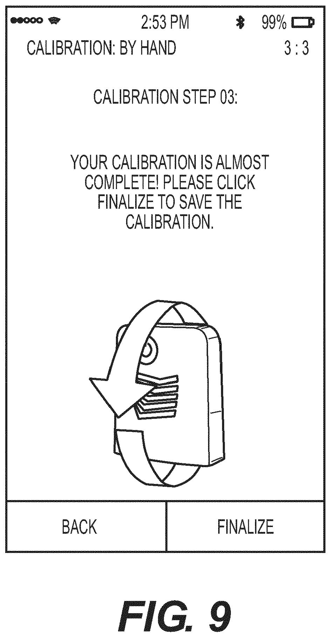

Post initiation of the calibration sequence, as depicted in FIG. 6, the user of the smart telephone is instructed to orient the Pod 270 in a particular way and thereafter rotate the Pod about a first axis perpendicular to a first of the planes. Again, readings are taken from all accelerometers during this rotation. The Pod 270 is then stopped, and thereafter rotated about a second axis perpendicular to a second of the planes as depicted in FIG. 7. Again, readings are taken from all accelerometers during this second rotation. The Pod is again stopped, and thereafter rotated about a third axis perpendicular to a third of the planes as depicted in FIG. 8. Again, readings are taken from all accelerometers during this third rotation. As depicted in FIG. 9, once the three rotation sequences have been completed, a finalize button becomes active on the smart telephone screen indicating that the calibration sequence has been successful. The outputs from the accelerometers at the multiple, fixed positions being recorded, on an accelerometer specific basis, are utilized to establish a zero acceleration reading for the applicable accelerometers. In addition to establishing zero with respect to the accelerometers, the calibration sequence may also map the local magnetic field and normalizes the output of the magnetometers to account for directional variance and the amount of distortion of the detected magnetic field.

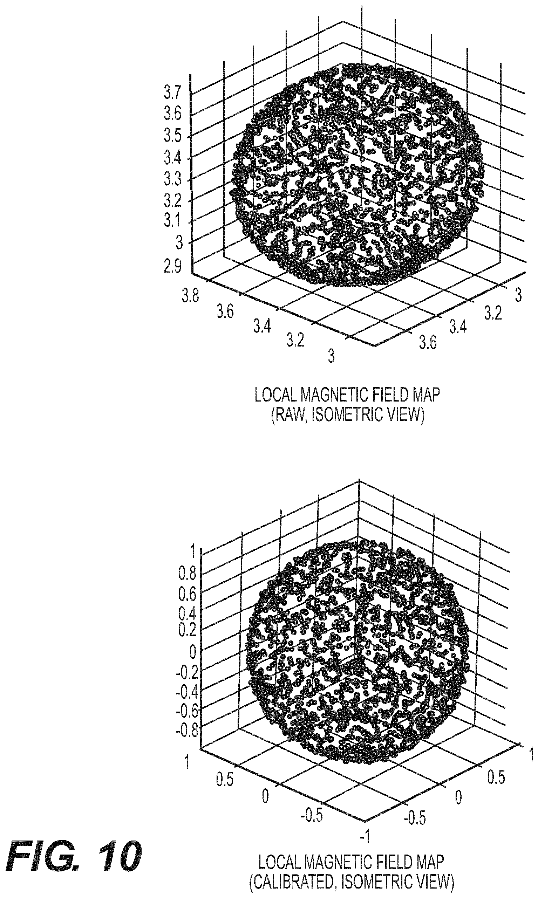

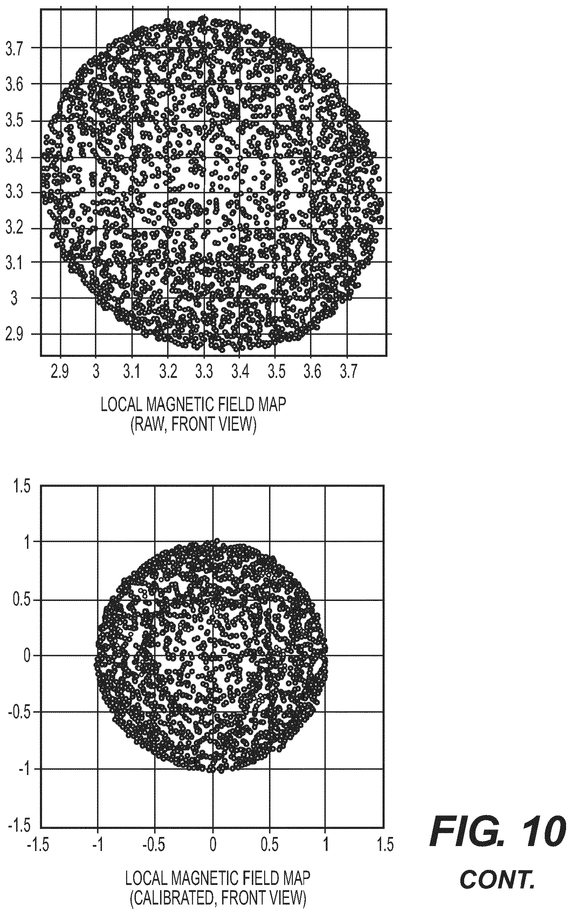

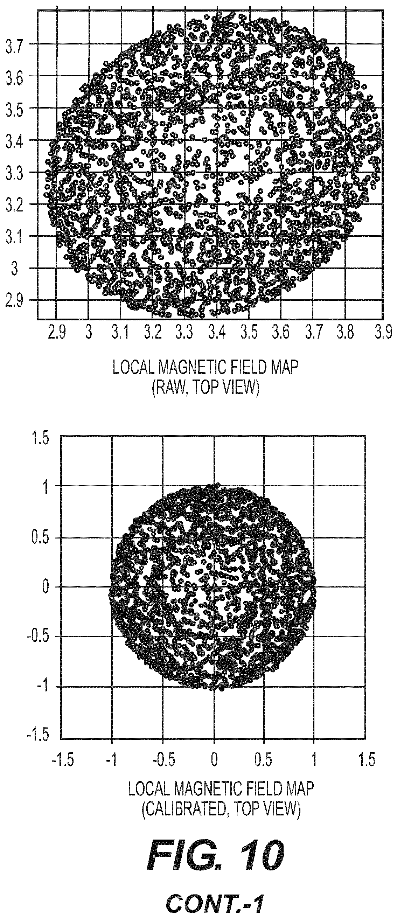

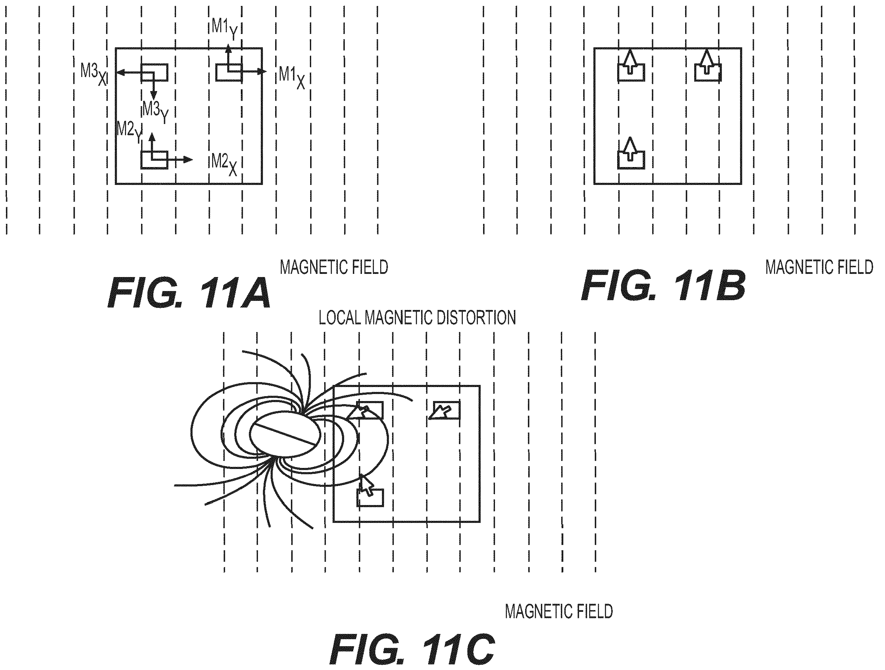

Referring to FIGS. 10 and 11, in order to map the local magnetic field for each magnetometer (presuming multiple magnetometers for each Pod 270 positioned in different locations), readings from the magnetometers are taken during the accelerometer calibration sequence previously described. Output data from each magnetometer is recorded so that repositioning of each magnetometer about the two perpendicular axes generates a point cloud or map of the three dimensional local magnetic field sensed by each magnetometer. FIG. 10 depicts an exemplary local magnetic field mapped from isometric, front, and top views based upon data received from a magnetometer while being concurrently rotated in two axes. As is reflected in the local magnetic field map, the local map embodies an ellipsoid. This ellipsoid shape is the result of distortions in the local magnetic field caused by the presence of ferrous or magnetic material, commonly referred to as hard and soft iron distortion. Soft iron distortion examples are materials that have low magnetic permeability, such as carbon steel, stainless steel, etc. Hard iron distortion is caused by material such as permanent magnets.

It is presumed that but for distortions in the local magnetic field, the local magnetic field map would be spherical. Consequently, the calibration sequence is operative to collect sufficient data point to describe the local magnetic field in different orientations by manual manipulation of the Pods 270. A calibration algorithm calculates the correction factors to map the distorted elliptic local magnetic field into a uniform spherical field.

Referencing FIG. 11, the multiple magnetometers positioned in different locations with respect to one another as part of a Pod 270 are used to detect local magnetic fields after the calibration is complete. Absent any distortion in the magnetic field, each of the magnetometers should provide data indicative of the exact same direction, such as polar north. But distortions in the local magnetic field, such as the presence of ferrous or magnetic materials (e.g. surgical instruments), causes the magnetometers to provide different data as to the direction of polar north. In other words, if the outputs from the magnetometers are not uniform to reflect polar north, a distortion has occurred and the Pod 270 may temporary disable the tracking algorithm from using the magnetometer data. It may also alert the user that distortion has been detected.

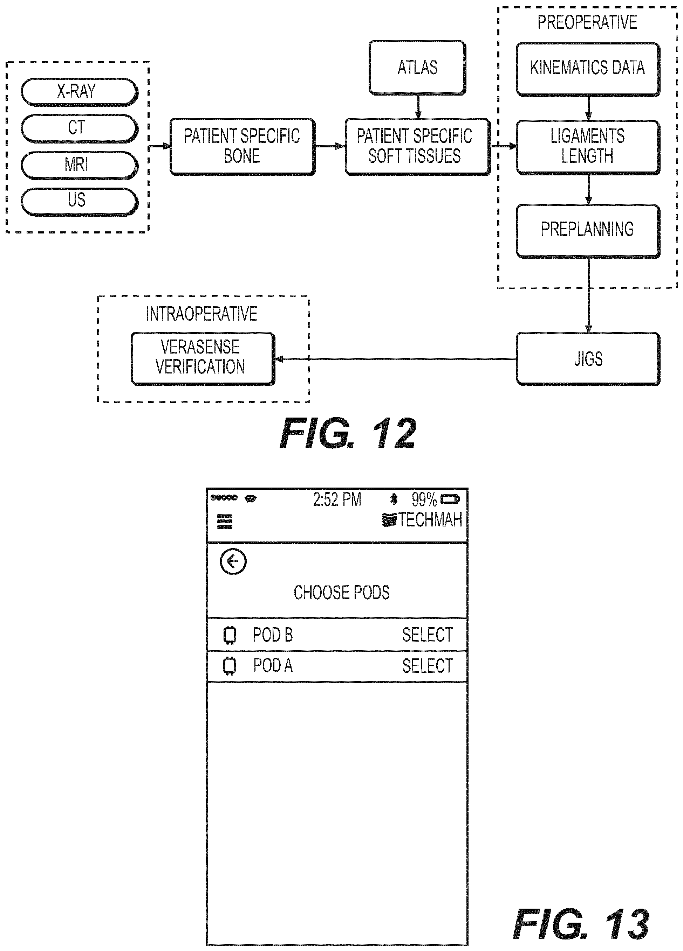

Referring to FIG. 12, an exemplary system and process overview is depicted for kinematic tracking of bones and soft tissues using IMUs or Pods 270 that makes use of a computer and associated software. For example, this kinematic tracking may provide useful information as to patient kinematics for use in preoperative surgical planning. By way of exemplary explanation, the instant system and methods will be described in the context of tracking bone motion and obtaining resulting soft tissue motion from 3D virtual models integrating bones and soft tissue. Those skilled in the art should realize that the instant system and methods are applicable to any bone, soft tissue, or kinematic tracking endeavor. Moreover, while discussing bone and soft tissue kinematic tracking in the context of the knee joint or spine, those skilled in the art should understand that the exemplary system and methods are applicable to joints besides the knee and bones other than vertebrae.

As a prefatory step to discussing the exemplary system and methods for use with bone and soft tissue kinematic tracking, it is presumed that the patient's anatomy (to be tracked) has been imaged (including, but not limited to, X-ray, CT, MRI, and ultrasound) and virtual 3D models of the patient's anatomy have been generated by the software pursuant to those processes described in the prior "Full Anatomy Reconstruction" section. Consequently, a detailed discussion of utilizing patient images to generate virtual 3D models of the patient's anatomy has been omitted in furtherance of brevity.

If soft tissue (e.g., ligaments, tendons, etc) images are available based upon the imaging modality, these images are also included and segmented by the software when the bone(s) is/are segmented to form a virtual 3D model of the patient's anatomy. If soft tissue images are unavailable from the imaging modality, the 3D virtual model of the bone moves on to a patient-specific soft tissue addition process. In particular, a statistical atlas may be utilized for estimating soft tissue locations relative to each bone shape of the 3D bone model.

The 3D bone model (whether or not soft tissue is part of the model) is subjected to an automatic landmarking process carried out by the software. The automatic landmarking process utilizes inputs from the statistical atlas (e.g., regions likely to contain a specific landmark) and local geometrical analyses to calculate anatomical landmarks for each instance of anatomy within the statistical atlas as discussed previously herein. In those instances where soft tissue is absent from the 3D bone model, the anatomical landmarks calculated by the software for the 3D bone model are utilized to provide the most likely locations of soft tissue, as well as the most likely dimensions of the soft tissue, which are both incorporated into the 3D bone model to create a quasi-patient-specific 3D bone and soft tissue model. In either instance, the anatomical landmarks and the 3D bone and soft tissue model are viewable and manipulatable using a user interface for the software (i.e., software interface).

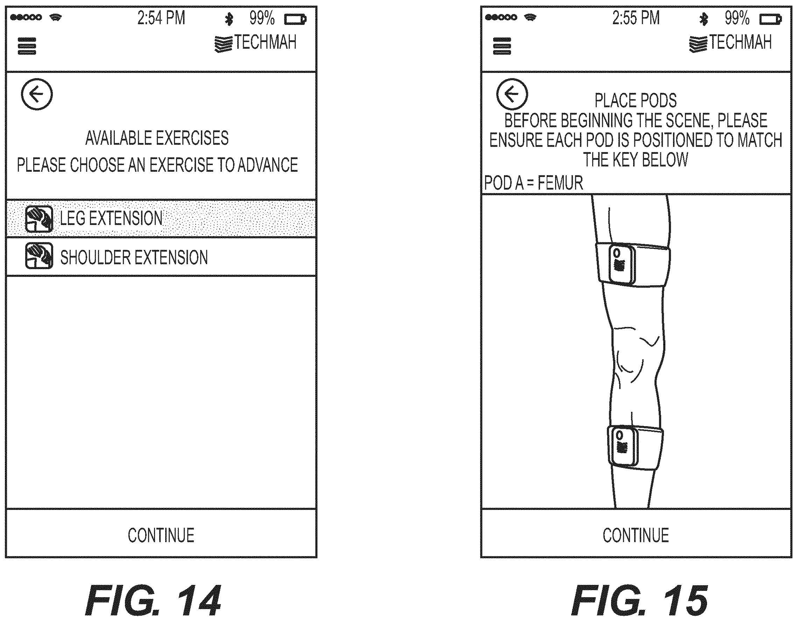

Referencing FIGS. 14-17, the exemplary software interface may comprise the patient portal 210 and be run on any processor based device including, without limitation, a desktop computer, a laptop computer, a server, a tablet computer, and a smart telephone. For purposes of explanation only, the device running the patient portal 210 will be described as a smart telephone. As shown specifically in FIG. 13, the anatomical tracking sequence may include a selection window on the data acquisition device that provides for selection of the Pods 270 that will be used to track the patient anatomy. Post selection of the Pods 270 that will be utilized, as shown in FIG. 14, the data acquisition device displays an additional window asking the user about the motion or exercise the patient will perform while being tracked. In this case, two exemplary motions are available for selection that include leg extension and arm extension. It should be realized that any number of programmed motion sequences may be programmed and available for selection as part of the exemplary patient portal 210.

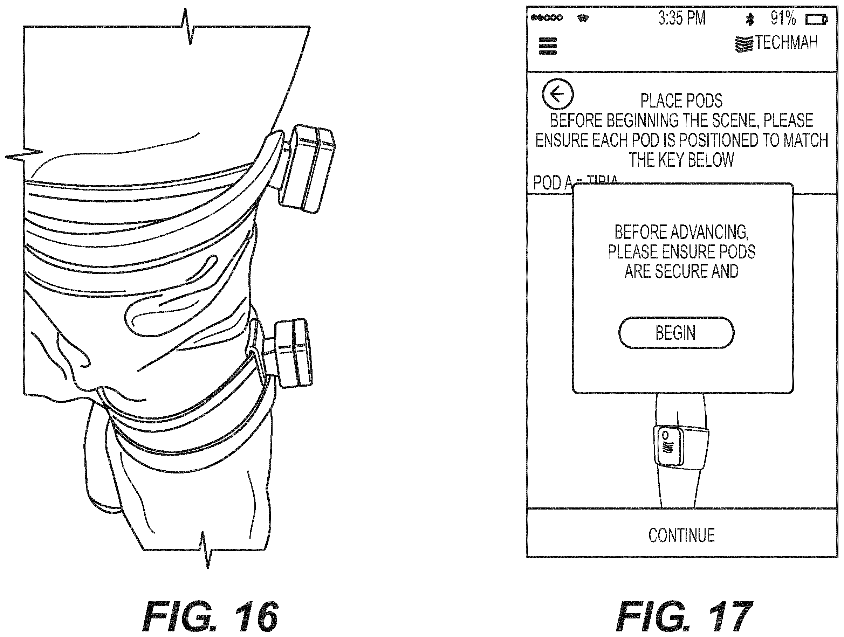

Based upon the motion sequence selected in FIG. 14, the data capture device running the patient portal 210 provides a visual indication to the user or patient instructing them as to the placement of the Pods 270 with respect to the patient as shown in FIG. 15. Consistent with this visual guidance, the patient dons a strap or other fixture in order to mount each Pod 270 as previously instructed, thereby resulting in the Pod placement on the patient as depicted in FIG. 16. Before initiating the motion sequence, the data acquisition device prompts the user, as shown in FIG. 17, to ensure the Pods 270 are secured and in the correct location. When the location of the Pods and mounting has been confirmed, the user selects the "BEGIN" button on the data acquisition device to initiate the data tracking by the Pods 270.

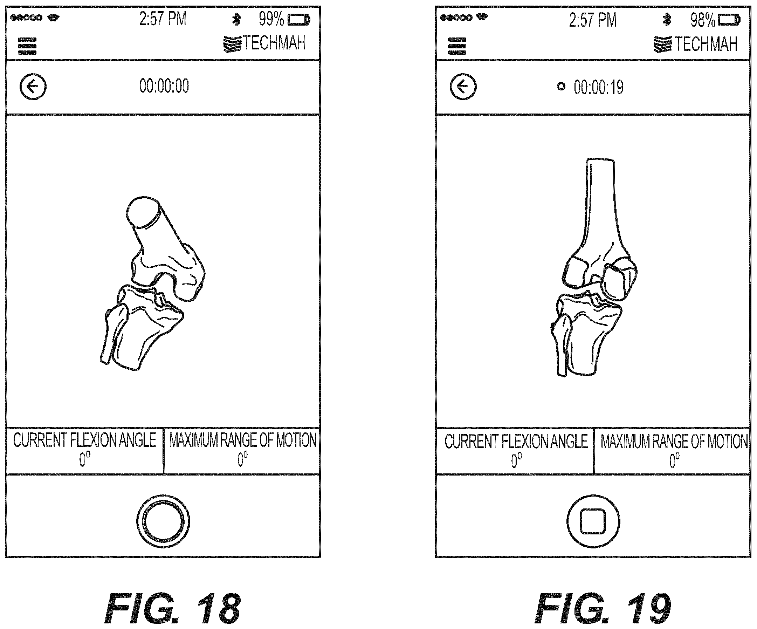

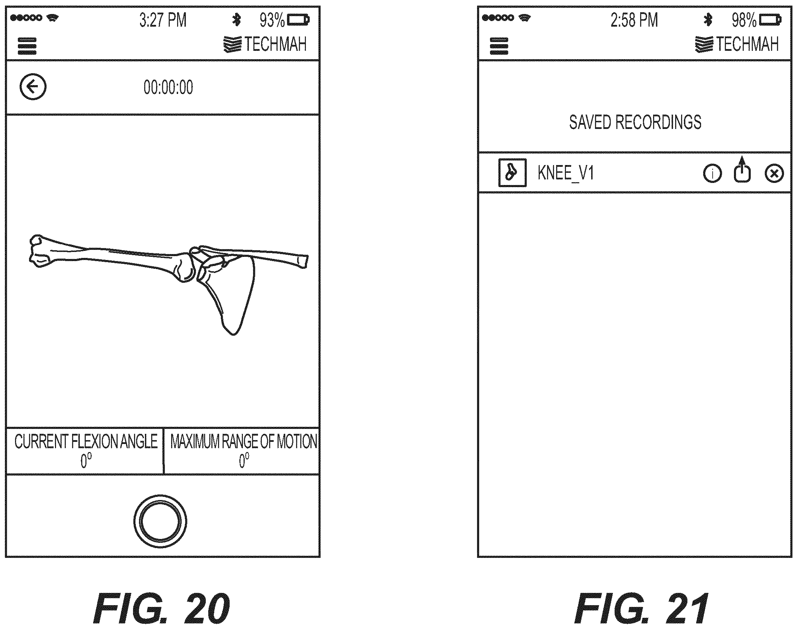

As shown in FIGS. 18-20, the software interface is communicatively coupled to the visual display of the data acquisition device that provides information to a user regarding the relative dynamic positions of the patient's bones and soft tissues that comprise the virtual bone and soft tissue model. In order to provide this dynamic visual information, which is updated in real-time as the patient's bones and soft tissue are repositioned based upon receiving orientation and position data from the IMUs or Pods 270. By way of example, the bones may comprise the tibia and femur in the context of the knee joint (see FIGS. 18, 19), or may comprise one or more vertebrae (e.g., the L1 and L5 vertebrae) in the context of the spine, or may comprise one or more bones associated with the shoulder joint (see FIG. 20). In order to track translation of the bones, additional tracking sensors (such as ultra-wide band) may be associated with each IMU (or combined as part of a single device) in order to register the location of each IMU with respect to the corresponding bone it is mounted to. In this fashion, by tracking the tracking sensors dynamically in 3D space and knowing the position of the tracking sensors with respect to the IMUs, as well as the position of each IMU mounted to a corresponding bone, the system is initially able to correlate the dynamic motion of the tracking sensors to the dynamic position of the bones in question. In order to obtain meaningful data from the IMUs, the patient's bones need to be registered with respect to the virtual 3D bone and soft tissue model. In order to accomplish this, the patient's joint or bone is held stationary in a predetermined position that corresponds with a position of the virtual 3D bone model. For instance, the patient's femur and tibia may be straightened so that the lower leg is in line with the upper leg while the 3D virtual bone model also embodies a position where the femur and tibia are longitudinally aligned. Likewise, the patient's femur and tibia may be oriented perpendicular to one another and held in this position while the 3D virtual bone and soft tissue model is oriented to have the femur and tibia perpendicular to one another. Using the UWB tracking sensors, the position of the bones with respect to one another is registered with respect to the virtual 3D bone and soft tissue model, as are the IMUs. It should be noted that, in accordance with the foregoing disclosure, the IMUs are calibrated prior to registration using the exemplary calibration sequence disclosed previously herein.

For instance, in the context of a knee joint where the 3D virtual bone and soft tissue model includes the femur, tibia, and associated soft tissues of the knee j oint, the 3D virtual model may take on a position where the femur and tibia lie along a common axis (i.e., common axis pose). In order to register the patient to this common axis pose, the patient is outfitted with the IMUs and tracking sensors (rigidly fixed to the tibia and femur) and assumes a straight leg position that results in the femur and tibia being aligned along a common axis. This position is kept until the software interface confirms that the position of the IMUs and sensors is relatively unchanged and a user of the software interface indicates that the registration pose is being assumed. This process may be repeated for other poses in order to register the 3D virtual model with the IMUs and tracking sensors. Those skilled in the art will understand that the precision of the registration will generally be increased as the number of registration poses increases.

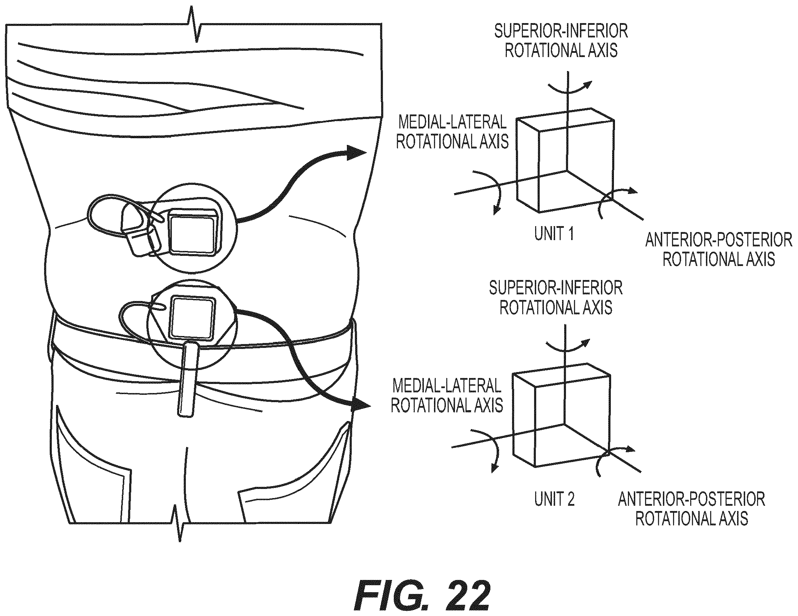

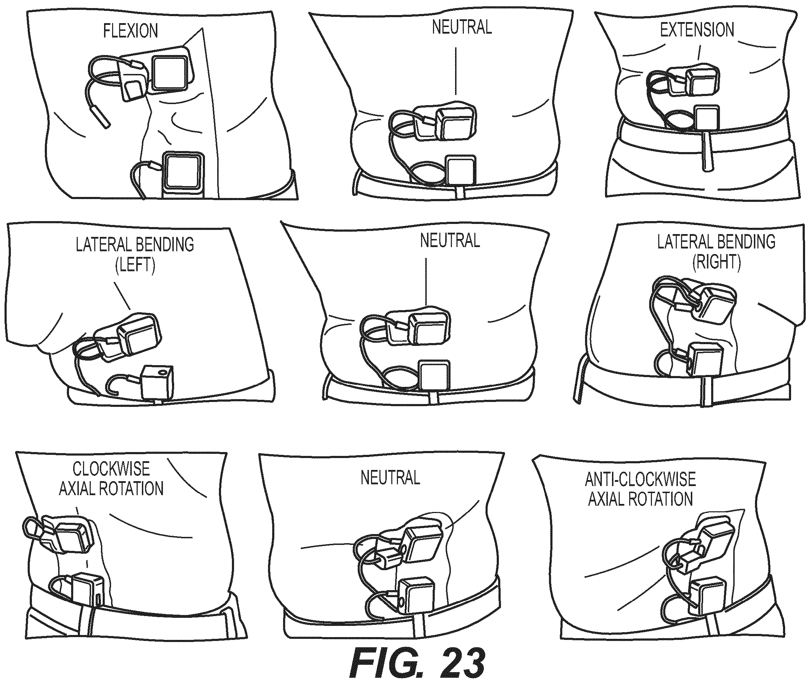

Referring to FIGS. 22 and 23, in the context of the spine where the 3D virtual model includes certain vertebrae of the spine, the 3D virtual model may take on a position where the vertebrae lie along a common axis (i.e., common axis pose) in the case of a patient lying flat on a table or standing upright. In order to register the patient to this common axis pose, the patient is outfitted with the IMUs or Pods 270 and other tracking sensors rigidly fixed in position with respect to the L1 and L5 vertebrae as depicted in FIG. 22, and assumes a neutral upstanding spinal position that correlates with a neutral upstanding spinal position of the 3D virtual model. This position is kept until the software interface confirms that the position of the IMUs and tracking sensors is relatively unchanged and a user of the software interface indicates that the registration pose is being assumed. This process may be repeated for other poses in order to register the 3D virtual model with the IMUs or Pods 270. Those skilled in the art will understand that the precision of the registration will generally be increased as the number of registration poses increases.

After registration, the patient anatomy may be moved in 3D space and dynamically tracked using the IMUs and tracking sensors so that the movement of the bones and soft tissue appears graphically on the visual display by way of movement of the 3D virtual model (see FIG. 23 in the context of the spine). While the patient moves, the software reads outputs from the IMUs and/or tracking sensors and processes these outputs to convert the outputs into dynamic graphical changes in the 3D model being depicted on the visual display (while keeping track of ligament length, joint pose and articulating surface contact areas, for example). The tracked motion of the patient's anatomy is dynamically updated on the data acquisition device and displayed dynamically so that the anatomical model moves in real-time as the patient moves consistent with the patient motion. This dynamic model motion may be recorded and saved as a separate motion file for transmission to the central repository 120 (and accessible to physicians and others having the requisite permission). Likewise, the dynamic motion may be saved as a file local to the data acquisition device to be played back later by a physician or therapist as a means to evaluate the motion (see FIG. 21). FIG. 18 depicts a virtual model of a patient's knee joint at the inception of the tracked motion, while FIG. 19 depicts the position of the knee joint nineteen seconds into the tracked motion sequence. Similarly, FIG. 20 depicts a virtual model of a patient's shoulder joint at the inception of the tracked motion.

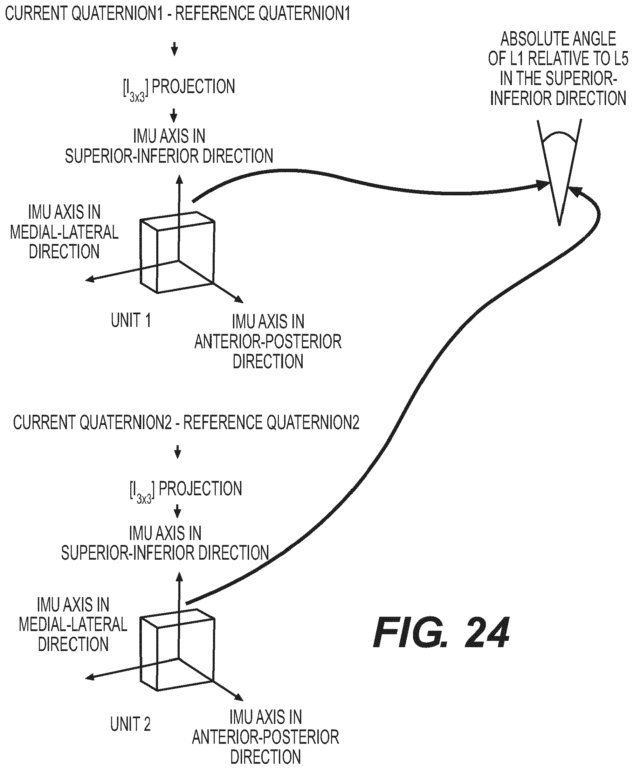

As shown in FIG. 24, when two or more IMUs are utilized to track a patient anatomy (e.g., a bone), the software interface determines the relative orientation of a first IMU with respect to a second IMU as discussed previously herein as each IMU processor is programmed to utilize a sequential Monte Carlo method (SMC) with von Mises-Fisher density algorithm to calculate changes in position of the IMUs or Pods 270 based upon inputs from the IMU's gyroscopes, accelerometers, and magnetometers.

The motion profile of healthy and pathological lumbar patients differ significantly, such that the out of plane motion is higher for pathological patients. Specifically, healthy and pathological can be differentiated using IMUs by having the patient perform three activities - axial rotation (AR), lateral bending (LB) and flexion-extension (FE). The coefficients for each of the prescribed motions are calculated as:

$$C_{FE} = \frac{A_{AR} + A_{LB}}{A_{FE}} \qquad C_{LB} = \frac{A_{AR} + A_{FE}}{A_{LB}} \qquad C_{AR} = \frac{A_{LB} + A_{FE}}{A_{AR}}$$

where $A_M$ represents the sum of the absolute value of angular motion, during motion M, for which C is calculated. By using IMUs or Pods 270, the exemplary system allows patient kinematic analysis and quantitative evaluation without the need for more expensive and intrusive tracking systems.

In exemplary form, the software of the patient portal 210 may also be able to calculate predicted load distribution upon the proximal tibia based upon kinematic data. In other words, in the context of a knee joint, the software tracks the movement of the distal femur and proximal tibia and records the frequency by which certain portions of the tibia surface are contacted by the distal femur through a range of motion of the knee joint. Based upon the frequency of contact between areas of the femur and tibia, the software is operative to generate color gradients reflective of the contact distribution so that areas in darker red are contacted the most frequent, whereas areas in blue are contacted the least, with gradients of shades between red and blue (including orange, yellow, green, and aqua) indicating areas of contact between the most and least frequent. By way of further example, the patient portal 210 may also highlight locations of soft tissue deformity as well as tracking anatomical axes through this range of motion.

For example, the patient portal 210 may utilize the location of soft tissue attachment sites stored in the statistical anatomical atlas to approximate the attachment sites and, based upon the kinematic movements of the tracked bones (in this case a femur and tibia), incorporates soft tissue data as part of the virtual models. More specifically, the software interface is communicatively coupled to a kinematic database and an anatomical database (e.g., a statistical bone atlas). Data from the two databases having been previously correlated (to link kinematic motion of bones with respect to one another with the locations of soft tissue attachment sites) allows the software to concurrently display anatomical data and kinematic data. Accordingly, the software is operative to include a ligament construction or reconstruction feature so that ligaments may be shown coupled to the bones. Likewise, the software interface tracks and records the motion of the bone and ligament model to show how the ligaments are stretched dynamically as the patient's bones are moved through a range of motion in a time lapsed sense. This range of motion data provides clearer images in comparison to fluoroscopy and also avoids subjecting the patient is harmful radiation.

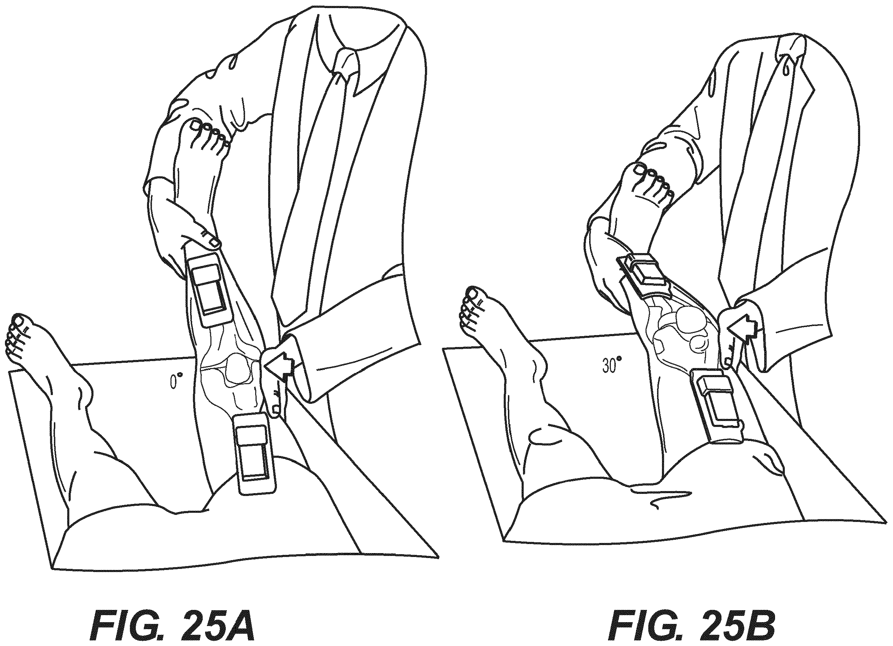

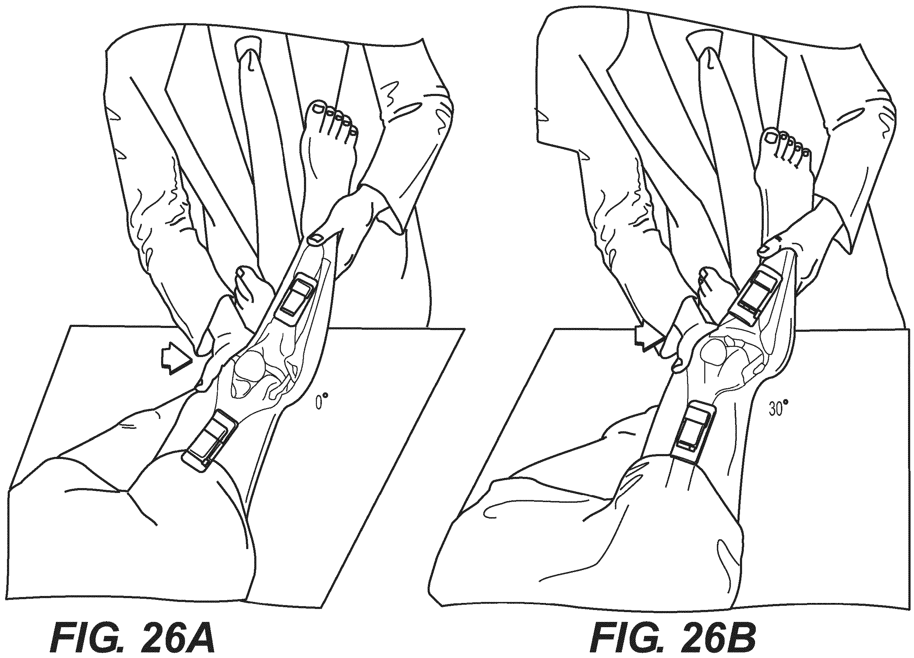

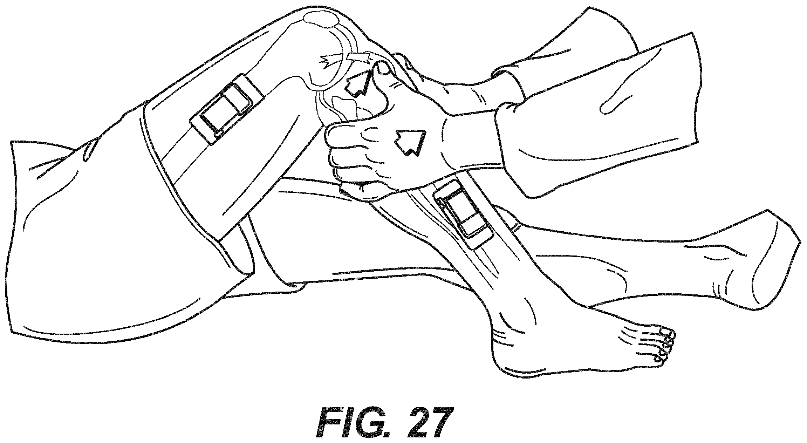

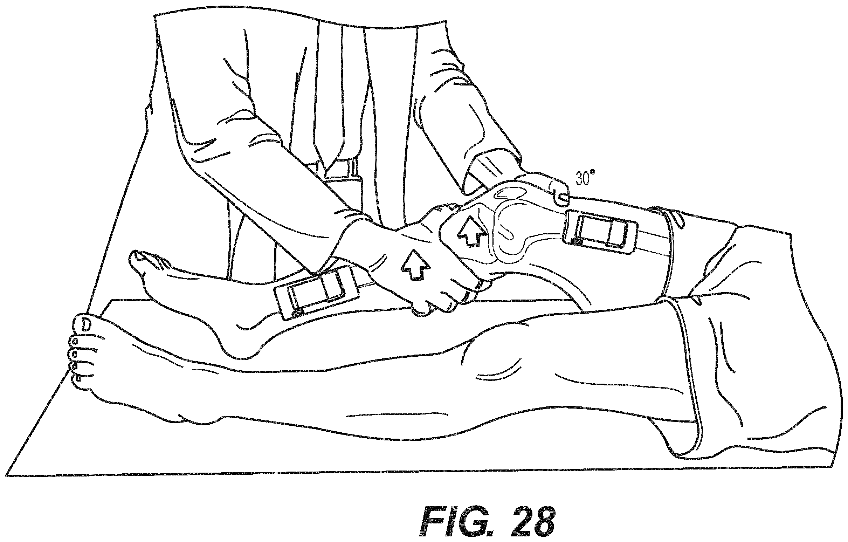

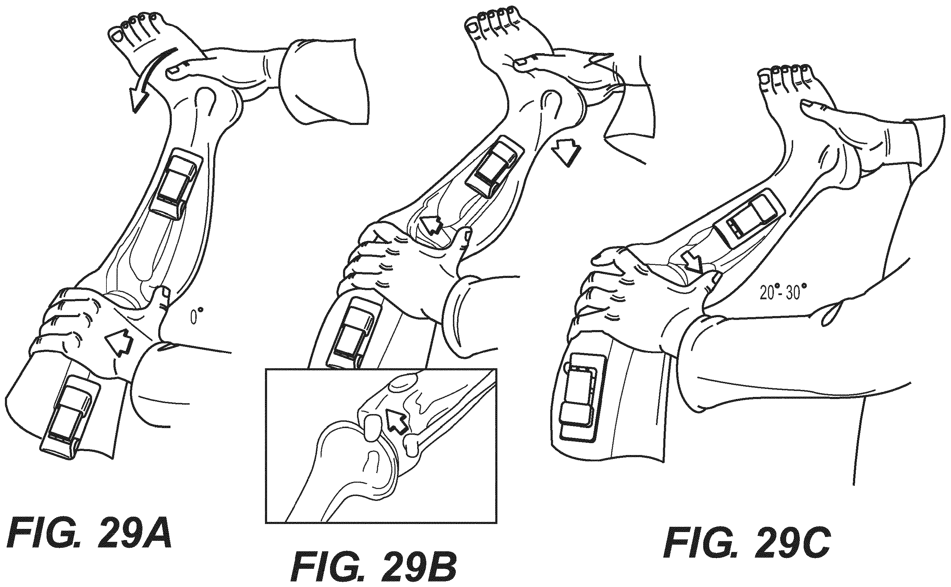

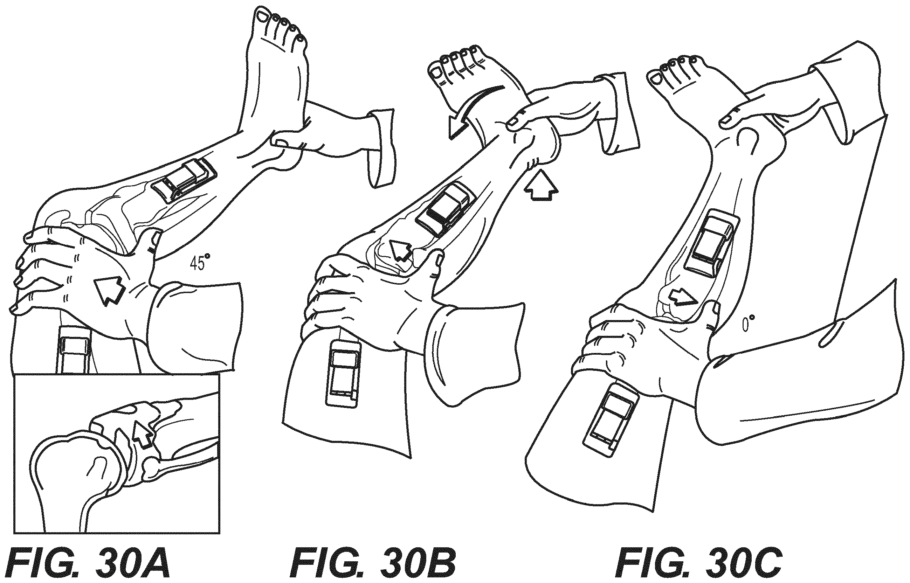

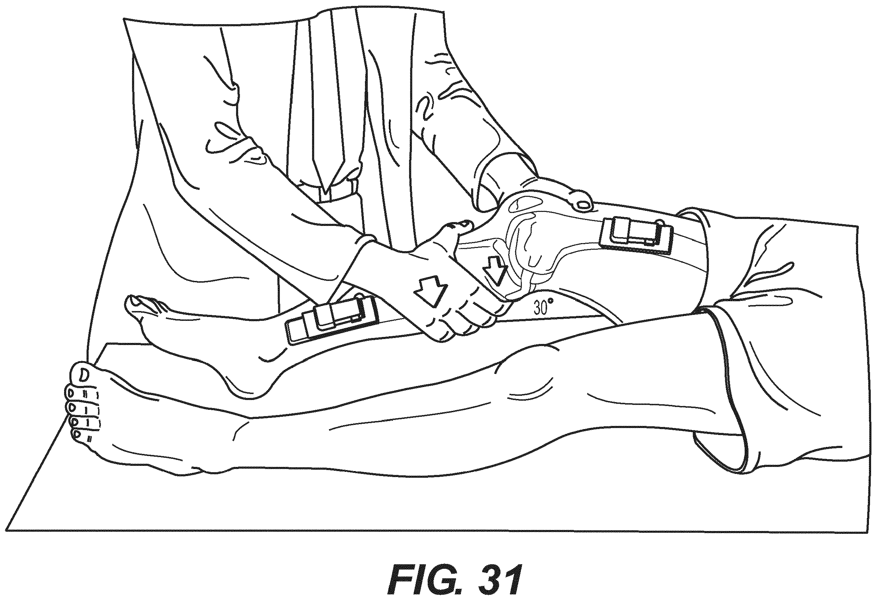

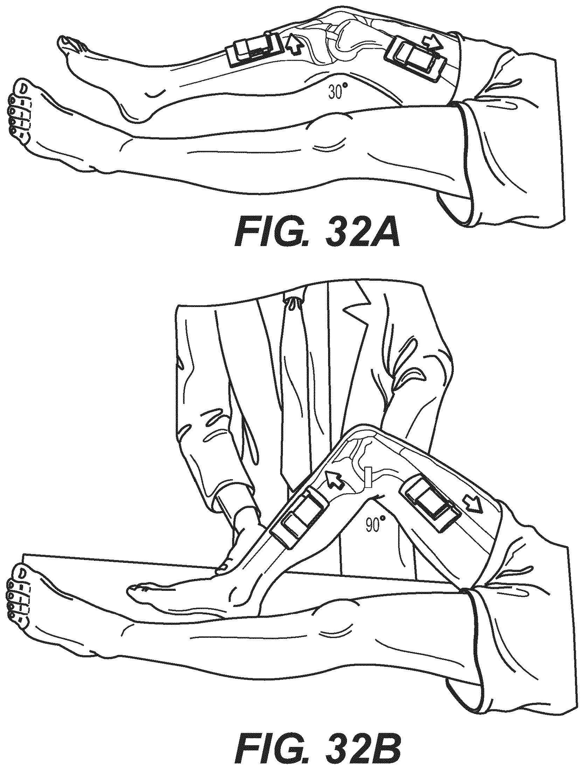

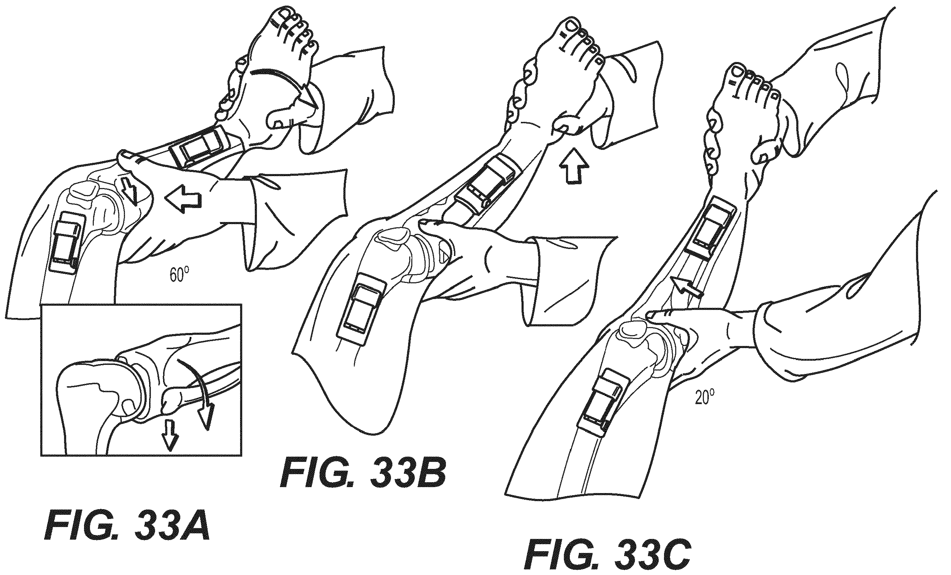

Referencing FIGS. 25-33, the visual representation of the 3D virtual bone and soft tissue model moving dynamically has particular applicability for a clinician performing diagnosis and pre-operative planning. For instance, the clinician may perform various tests on a knee joint, such as the drawer test, to view movement of the bone and soft tissue across a range of motion. This kinematic tracking information may be imported into a surgical planning interface, for example, to restrict resection plans that may violate the ligament lengths obtained from the kinematic data. Kinematic data may also be used for real time quantification of various knee tests (e.g., Oxford knee score) or for the creation of novel quantifiable knee scoring systems using statistical pattern recognition or machine learning techniques. In sum, the clinician testing may be used for more accurate pre-operative and post-operative evaluations when alternatives, such as fluoroscopy, may be more costly and more detrimental to patient wellness.

In exemplary form, each Pod 270 includes at least one IMU and an associated power supply, IMU processor, and a wireless transmitter, in addition to a power on-off switch. In this fashion, each Pod 270 is a self-contained item that is able to be coupled to a patient's anatomy to track the anatomy or anatomical feature and then be removed. In the context of reuse and sterilization, each Pod 270 may be reusable or disposable.

While the exemplary Pods 270 have been described as having IMUs and optionally UWB electronics, the following description pertains to Pods 270 that in fact include UWB electronics and exemplary uses for these Pods.

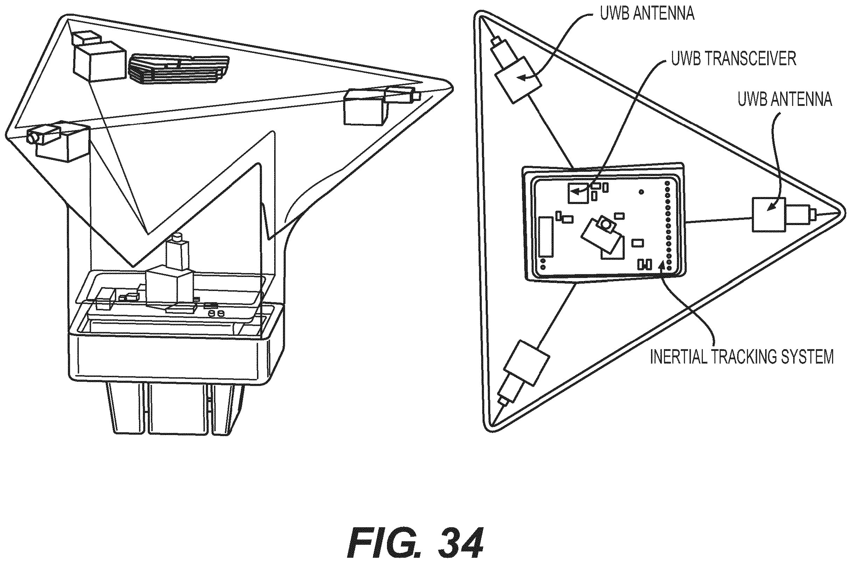

Referring to FIGS. 34-48, an exemplary Pod 270 may make use of ultra wide band (UWB) and inertial measurement units (IMUs) and comprises at least one central unit (i.e., a core unit) and one peripheral unit (i.e., a satellite unit). Each central unit comprises, in exemplary form, at least one microcomputer, at least one tri-axial accelerometer, at least one tri-axial gyroscope, at least three tri-axial magnetometers, at least one communication module, at least one UWB transceiver, at least one multiplexer, and at least four UWB antennas (see FIG. 34) Also, each peripheral unit comprises, in exemplary form, at least one microcomputer, at least one tri-axial accelerometer, at least one tri-axial gyroscope, at least three tri-axial magnetometers, at least one communication module, at least one UWB transceiver, at least one multiplexer, and at least four UWB antennas.

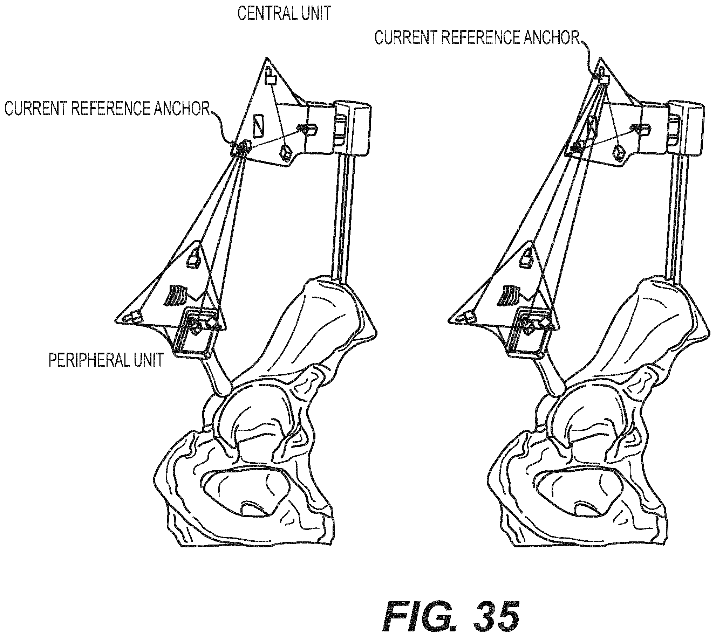

As shown in FIGS. 35A, 35B, this exemplary system making use of the hybrid UWB and IMU surgical navigation system uses the central unit as a positional reference, and navigate the relative translations and orientations of the surgical instrument using the peripheral unit.

One of the important aspects of using an UWB navigation system for high accuracy surgical navigation is to account for antenna phase center variation at the transmitters and receivers. Ideally all frequencies contained in the pulse are radiated from the same point of the UWB antenna and, thus, would have a fixed phase center. In practice, the phase center varies with both frequency and direction. UWB antenna phase centers can vary by up to 3 centimeters as the angle of arrival is varied.

In order to mitigate antenna phase center error, each UWB antenna should have its phase center precisely characterized at all possible angles of arrival over the entire operational frequency band. Phase center characterization and mitigation is routinely performed in GPS systems to improve location accuracy. UWB tags and anchors can utilize a variety of UWB antennas including monopoles, dipoles, spiral slots, and Vivaldis.

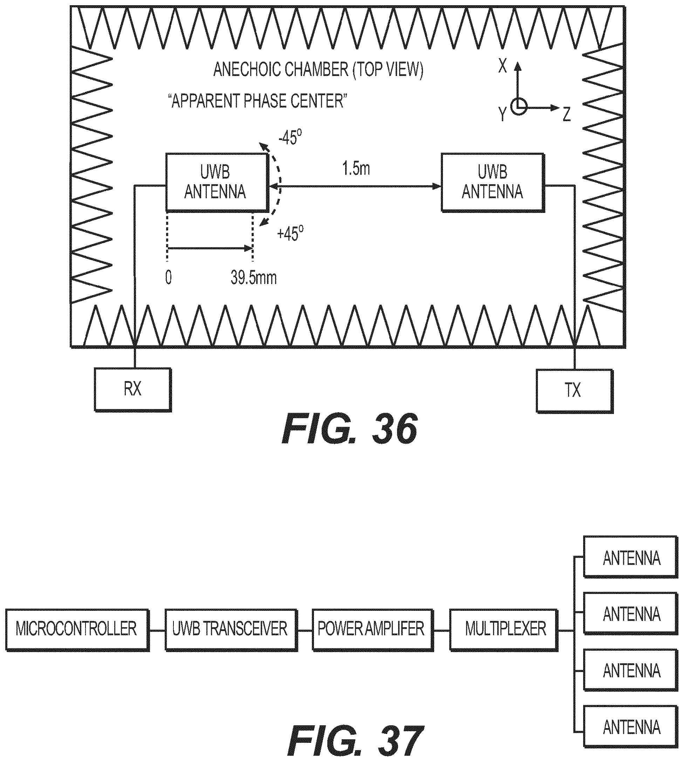

FIG. 36 outline how a UWB antenna phase center can be characterized in 3-D so that the phase center bias can subsequently be removed during system operation. The UWB antenna is placed in an anechoic chamber to quantify how the phase center is affected by the directivity based on time domain measurements. Two of the same UWB antennas are put face to face and separated by a distance of 1.5 meters. The receiving antenna is rotated around the calculated "apparent phase center" from -45 to 45 degrees at 5 degrees per step. The apparent phase center is tracked on the UWB receiving antenna as it is rotated from -45 to 45 degrees with an optically tracked probe. The optical system provides a ground truth reference frame with sub-millimeter accuracy. These reference points from the optical system are used to calculate the actual center of rotation during the experiment. This allows changes in the actual phase center as the receiving antenna is rotated to be separated from physical movement of the apparent phase center.

This process is used to characterize the UWB antenna phase center variation for each UWB antenna design used in the UWB navigation system (e.g., monopole, spiral slot). Once the UWB antenna phase center has been fully characterized in 3-D for all possible angles of arrival, the phase center error can be removed from the system by subtracting out the phase center bias for each tag using the calculated 3-D position of each tag.

An alternative approach for removing phase center bias is to rigidly attach the antenna to a motorized gimbal where a digital goniometer or inertial measurement unit can provide the angular feedback to a control system of the motors so that the antenna can be positioned and orientated in its optimal positions.

As shown in FIG. 37, by connecting multiple antennas to a single transceiver, it enables one to create multiple anchors or tags within the same UWB unit. The UWB antenna array in both central and peripheral units can be arranged in any configuration with the condition that one of the antennas does not reside on the same plane with the other three. For example, a tetrahedron configuration will satisfy this condition.

The UWB antenna array in the central unit serves as the anchors for the system. For example, a tetrahedron configuration will have four antennas connected to a single UWB transceiver. This creates four anchors in the central unit. With a single clock, and a single transceiver to feed the UWB pulses into multiple antennas, this configuration enables clock synchronization among all anchors in the unit. This configuration can tremendously improve the flexibility of the installation of the anchors, as well as easing the calibration procedure of the unit. In a short range localization application, a single central system is sufficient to provide adequate anchors for localization. In a large area localization application, multiple central systems can be used. The clocks of the central units are synchronized during operation with either wired or wireless methods.

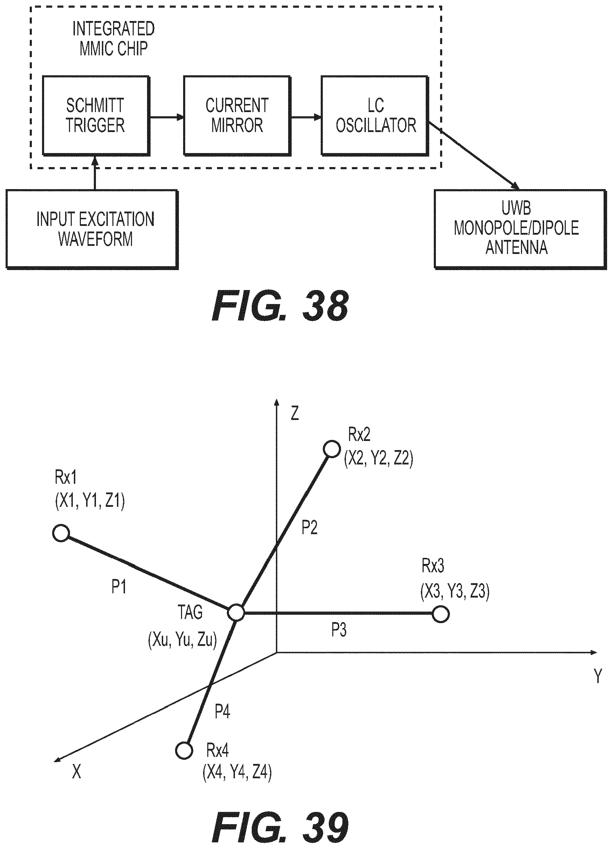

Referring to FIG. 37, a block diagram of the silicon-germanium monolithic microwave intergrated circuit (MMIC) based UWB transmitter is depicted where a crosscoupled oscillator core is transiently turned on by a current spike generated by a Schmitt trigger driving a current mirror. FIG. 38 depicts an integrated board design with the MMIC at the feed point of the UWB antenna. The MMIC based transmitter is more compact and only has a load requirement of 6 milliwatts for operation (1.5 volts, 4 milliamps).

The UWB antenna array in the peripheral unit serves as the tags for the system. For example, a tetrahedron configuration has four antennas connected to a single UWB transceiver. This creates four tags in the peripheral unit. With a single clock, and a single transceiver to feed the UWB pulses into multiple antennas, this configuration enables clock synchronization among all anchors in the unit. This configuration enables the ability to calculate orientations of a peripheral unit by applying rigid body mechanics based on the localization of the tags.

Referring to FIG. 39, localization of the tag is achieved with a TDOA algorithm, which looks at the relative time differences between the anchors. There are four anchors at known positions $R_{x1}$ or $(x_1, y_1, z_1)$, $R_{x2}$ or $(x_2, y_2, z_2)$, $R_{x3}$ or $(x_3, y_3, z_3)$, and $R_{x4}$ or $(x_4, y_4, z_4)$, and a tag at an unknown position $(x_u, y_u, z_u)$. The measured distance between the four known position receivers and the unknown position tag can be represented as $\rho_1$, $\rho_2$, $\rho_3$, and $\rho_4$, which is given by:

$$\rho_i = \sqrt{(x_i - x_u)^2 + (y_i - y_u)^2 + (z_i - z_u)^2} + ct_u$$
$$= f(x_u, y_u, z_u, t_u) \tag{1}$$

where i = 1, 2, 3, and 4, c is speed of light, and $t_u$ is the unknown time delay in hardware. The differential distances between four anchors and the tag can be written as

$$\Delta\rho_{1k} = \rho_1 - \rho_k$$
$$= \sqrt{(x_1 - x_u)^2 + (y_1 - y_u)^2 + (z_1 - z_u)^2} \tag{2}$$
$$- \sqrt{(x_k - x_u)^2 + (y_k - y_u)^2 + (z_k - z_u)^2}$$

where $k$ = 2, 3, and 4, and the time delay $t_u$ in hardware has been cancelled. Differentiating this equation will give

$$d\Delta\rho_{1k} = \frac{(x_1 - x_u)dx_u + (y_1 - y_u)dy_u + (z_1 - z_u)dz_u}{\sqrt{(x_1 - x_u)^2 + (y_1 - y_u)^2 + (z_1 - z_u)^2}}$$
$$+ \frac{(x_k - x_u)dx_u + (y_k - y_u)dy_u + (z_k - z_u)dz_u}{\sqrt{(x_k - x_u)^2 + (y_k - y_u)^2 + (z_k - z_u)^2}}$$
$$= \left(\frac{x_1 - x_u}{\rho_1 - c\tau_u} + \frac{x_k - x_u}{\rho_k - c\tau_u}\right)dx_u \tag{3}$$
$$+ \left(\frac{y_1 - y_u}{\rho_1 - c\tau_u} + \frac{y_k - y_u}{\rho_k - c\tau_u}\right)dy_u$$
$$+ \left(\frac{z_1 - z_u}{\rho_1 - c\tau_u} + \frac{z_k - z_u}{\rho_k - c\tau_u}\right)dz_u$$

In equations (3-5), $x_u$, $y_u$, and $z_u$ are treated as known values by assuming some initial values for the tag position. $dx_u$, $dy_u$, and $dz_u$ are considered as the only unknowns. From the initial tag position the first set of $dx_u$, $dy_u$, and $dz_u$ can be calculated. These values are used to modify the tag position $x_u$, $y_u$, and $z_u$. The updated tag position $x_u$, $y_u$, and $z_u$ can be considered again as known quantities. The iterative process continues until the absolute values of $dx_u$, $dy_u$, and $dz_u$ are below a certain predetermined threshold given by

$$\varepsilon = \sqrt{dx_u^2 + dy_u^2 + dz_u^2} \tag{4}$$

The final values of $x_u$, $y_u$, and $z_u$ are the desired tag position. The matrix form expression of (5) is

$$\begin{bmatrix} d\Delta\rho_{12} \\ d\Delta\rho_{13} \\ d\Delta\rho_{14} \end{bmatrix} = \begin{bmatrix} \alpha_{11} & \alpha_{12} & \alpha_{13} \\ \alpha_{21} & \alpha_{22} & \alpha_{23} \\ \alpha_{31} & \alpha_{32} & \alpha_{33} \end{bmatrix} \begin{bmatrix} dx_u \\ dy_u \\ dz_u \end{bmatrix} \qquad (5)$$

where

$$\alpha_{k-1,1} = \frac{x_1 - x_u}{\rho_1 - c\tau_u} + \frac{x_k - x_u}{\rho_k - c\tau_u}$$

$$\alpha_{k-1,2} = \frac{y_1 - y_u}{\rho_1 - c\tau_u} + \frac{y_k - y_u}{\rho_k - c\tau_u} \qquad (6)$$

$$\alpha_{k-1,3} = \frac{z_1 - z_u}{\rho_1 - c\tau_u} + \frac{z_k - z_u}{\rho_k - c\tau_u}$$

The solution of equation (6) is given by

$$\begin{bmatrix} dx_u \\ dy_u \\ dz_u \end{bmatrix} = \begin{bmatrix} \alpha_{11} & \alpha_{12} & \alpha_{13} \\ \alpha_{21} & \alpha_{22} & \alpha_{23} \\ \alpha_{31} & \alpha_{32} & \alpha_{33} \end{bmatrix}^{-1} \begin{bmatrix} d\Delta\rho_{12} \\ d\Delta\rho_{13} \\ d\Delta\rho_{14} \end{bmatrix} \qquad (7)$$

where $[\,]^{-1}$ represents the inverse of the $\alpha$ matrix. If there are more than four anchors, the least-squares approach can be applied to find the tag position.

A proof of concept experiment was conducted to examine the translation tracking of the UWB system with a TDOA algorithm. An experiment was run using five anchors while tracking a single tag dynamically along a rail. An optical tracking system was used for comparison.

The operating room is a harsh indoor environment for UWB positioning. FIG. 199(A) shows a truncated list of parameters for the line-of-sight (LOS) operating room environment fit to the IEEE 802.15.4a channel model (shown in equation 8) that were obtained with time domain and frequency domain experimental data. A pathloss for the operating room (OR) environment may be obtained by fitting experimental data to equation 9 and compared to residential LOS, commercial LOS, and industrial LOS. The pathloss in the OR is most similar to residential LOS, although this can change depending on which instruments are placed near the transmitter and receiver or the locations of the UWB tags and anchors in the room.

$$h(t) = \sum_{l=0}^{L} \sum_{k=0}^{K} a_{k,l} \exp(j\varphi_{k,l}) \, \delta(t - T_l - \tau_{k,l}) \qquad (8)$$

$$PL(d) = PL_0 + 10n \, log_{10}\left(\frac{d}{d_0}\right) \qquad (9)$$

where equation 8 is the impulse response of the UWB channel in the time domain, and equation 9 is the pathloss model used in the corresponding UWB channel.

The orientations of the units can be estimated by using four tags attached rigidly on the same body. Given four set of points Z = {P1,P2,P3,P4}, which are moving as a single, whole rigid body relative to the anchors. The relative change in orientations between the tags and anchors can be calculated by minimizing the following equation,

$$\sum_{1}^{4} \|Z_i - T * Z_n\| \qquad (10)$$

18

where $Z_i = Z*T_i$, with $T_i$ being the initial orientations of the tags relative to the anchors, T is the new orientation to be calculated, and Zn is the new location of the points.

Apart from the localization capability, UWB can also significantly improve the wireless communication of the surgical navigation system. Preexising surgical navigation systems utilizing wireless technology are typically confined within the 400MHz, 900MHz, and 2.5GHz Industrial, Scientific, and Medical (ISM) band. The landscape of these bands are heavily polluted due to many other devices sharing the same band. Secondly, although the data rate in these bands vary with the protocol, it is becoming impossible to handle the increasing demand of larger data sets necessary for navigation systems. UWB technology can also serve as a communication device for the surgical navigation system. It operates in a relatively clean bandwidth and it has several folds higher data rate than the conventional wireless transmission protocol. In addition, the power consumption of UWB communication is similar to Bluetooth low energy (BLE).

Turning to the inertial navigation system of the present disclosure, this inertial navigation system uses the outputs from a combination of accelerometers, gyroscopes, and magnetometers to determine the translations and orientations of the unit. For translation navigation, the accelerometer provides linear accelerations experienced by the system. The translations of the system can be navigated using the dead reckoning method. Using the equation of motion, the basic calculation for position from the accelerometer data is to integrate acceleration over time twice as shown below,

$$v = \int a\Delta t = v_i + a\Delta t \tag{11}$$

$$s = \int v\Delta t = s_i + v_i\Delta t + \frac{1}{2}a\Delta t^2 \tag{12}$$

where $a$ is acceleration, v is velocity, $v_i$ is velocity of the previous state, $s$ is position, $s_i$ is position from the previous state, and $\Delta t$ is time interval.

Upon close examination, one will notice that the velocity and position from the previous states also contributes the calculation of the current states. In other words, if there is any noise and error from the previous states, it will be accumulated. This is known as the arithmetic drift error. A difficult part of designing the inertial navigation system is the ability to control and minimize this drift. In the present case, this drift is controlled by the UWB system, which is described in more detail hereafter.

For orientation navigation, a multitude of estimation and correction algorithms (e.g. Kalman filters, particle filters) can be used to perform sensor fusion. The fundamental of sensor fusion with an inertial device is to use gyroscopes to estimate the subsequent orientations of the unit and, at the same time, uses accelerometers and magnetometers to correct the error from a previous estimation. Different algorithms control the error correction in different ways. With a Kalman filter, the system is assumed to be linear and Gaussian, while no such assumption is made with a particle filter.

The basic Kalman filter can be separated into 2 major sets of equations, which are the time update equations and the measurement update equations. The time update equations predict the *priori* estimates at time *k* with the knowledge of the current states and error covariance at time *k-1* in equation (13) respectively.

$$x_k = Ax_{k-1} + Bu_{k-1} + w_{k-1} \tag{13}$$

$$P_k^- = AP_{k-1}A^T + Q \tag{14}$$

where $x_k$ is the state vector of the current state, $x_{k-1}$ is the state vector from the previous state, $A$ is the transitional matrix model to transform the previous state into the current state, $B$ is the matrix model for controlled input $u_{k-1}$ from the previous state, and $w_{k-1}$ is the process noise, which is independent and normally distributed around zero means with process noise covariance matrix $Q$.

The measurements update equations use the measurements acquired with the *priori* estimates to calculate the *posteriori* estimates.

$$S_k = HP_k^- H^T + R \tag{15}$$

$$K_k = P_k^- H_k^T S_k^{-1} \qquad (16)$$

$$\hat{x}_k = \hat{x}_k^- + K_k \tilde{y}_k, \qquad \tilde{y}_k = z_k - H \hat{x}_k^- \qquad (17)$$

$$P_k = (I - K_k H_k) P_k^- \qquad (18)$$

where $P_k^-$ is the *priori* error covariance matrix, $P_k$ is the *priori* error covariance matrix, $S_k$ is the innovation error covariance matrix, $H$ is the *priori* prediction, $\hat{x}_k$, is the *posteriori* state estimate, and $\hat{x}_k^-$ is the *priori* estimate, $K_k$ is the optimal Kalman gain, $z_k$ is the measurement.

The *posteriori* estimate is then use to predict *priori* estimate at the next time step. As displayed from the equations above, no further information is required beside the state and error covariance from the previous state. The algorithm is extremely efficient and suitable for the navigation problem where multiple concurrent input measurements are required.

There are multiple different implementations of a Kalman filter that tackles the linear and Gaussian assumptions such as an extended Kalman filter that linearize the system, as well as Sigma point and Unscented Kalman filters that provide non-linear transformation of the system.

The fundamental of the particle filter (PF) or Sequential Monte Carlo (SMC) filter is solving a probabilistic model that computes the posterior probability density function of an unknown process and uses it in the estimation calculation. It generally involves two-stage processes of state prediction and state update to resolve the posterior density. Using a particle filter can be considered a brute force approach to approximate the posterior density with a large sum of independent and identically distributed random variables or particles from the same probability density space.

Consider a set of $N$ independent random samples are drawn from a probability density $p(x_k|z_k)$,

$$x_k(i) \sim p(x_k|z_{1:k}), \qquad i = 1:N \qquad (19)$$

The Monte Carlo representation of the probability density can then be approximated as,

$$p(x_k|z_{1:k}) \approx \frac{1}{N} \sum_{i=1}^{N} \delta_{x_k(i)}(x_k) \qquad (20)$$

where $\delta_{x(i)}$ is the Dirac delta function of the points mass.

Using this interpretation, the expectation of the any testing function $h(x)$ is given by

$$\mathbb{E}(h(x_k)) = \int h(x_k) p(x_k|z_{1:k}) dx_k \approx \int h(x_k) \frac{1}{N} \sum_{i=1}^{N} \delta_{x_k(i)}(x_k) \, dx_k$$
$$= \frac{1}{N} \sum_{i=1}^{N} h(x_k(i)), \quad i = 1:N \qquad (21)$$

In practice, sampling from $p(x)$ directly is usually not possible due to latent hidden variables in the estimation. Alternatively, samples are drawn from a different probability density $q(x_k|z_{1:k})$ is proposed,

$$x_k(i) \sim q(x_k|z_{1:k}), \qquad i = 1:N \qquad (22)$$

which is generally known as the importance function or the importance density. A correction step is then used to ensure the expectation estimation from the probability density $q(x_k|z_{1:k})$ remains valid. The correction factor, which is generally regarded as the importance weights of the samples ($w_k(i)$), is proportional to the ratio between the target probability density and the proposed probability density,

$$w_k(i) \propto \frac{p(x_k|z_{1:k})}{q(x_k|z_{1:k})} \quad i = 1:N \tag{23}$$

The importance weights are normalized,

$$\sum_{i=1}^{N} w_k(i) = 1 \tag{24}$$

Based on the sample drawn from equation (22), the posterior probability density becomes,

$$p(x_k|z_{1:k}) = \frac{p(z_k|x_k|z_{k-1})p(x_k|z_{k-1})}{p(z_k|z_{k-1})} \tag{25}$$

$$= \frac{p(z_k|x_k)p(x_k|x_{k-1})}{p(z_k|z_{k-1})} p(x_k|z_{1:k-1}) \tag{26}$$

$$\propto p(z_k|x_k)p(x_k|x_{k-1})p(x_k|z_{1:k-1}) \tag{27}$$

And the importance weight from equation (22)(23) becomes,

$$w_k(i) \propto \frac{p(z_k|x_k(i))p(x_k(i)|x_{k-1}(i))p(x_{1:k-1}(i)|z_{1:k-1})}{q(x_k(i)|x_{1:k-1}(i))q(x_{1:k-1}(i)|z_{1:k-1})}, \quad i = 1:N \tag{28}$$

$$= w_{k-1}(i)\frac{p(z_k|x_k(i))p(x_k(i)|x_{k-1}(i))}{q(x_k(i)|x_{1:k-1}(i))} \tag{29}$$

$$\propto w_{k-1}(i)\frac{p(z_k|x_k(i))p(x_k(i)|x_{k-1}(i))}{q(x_k(i)|x_{k-1}(i))} \tag{30}$$

The posterior probability density can then be approximated empirically by,

$$p(x_k|z_{1:k}) \approx \sum_{i=1}^{N} w_k(i)\,\delta_{x_k(i)}(x_k) \tag{31}$$

The expectation of the estimation from equation (20) can be expressed as,

$$\mathbb{E}\big(h(x_k)\big) = \int h(x_k)p(x_k|z_{1:k})dx_k \approx \int h(x_k)\sum_{i=1}^{N} w_k(i)\,\delta_{x_k(i)}(x_k)$$
$$= \sum_{i=1}^{N} w_k(i)h(x_k(i)), \quad i = 1:N \tag{32}$$

The technique demonstrated by equations (28-31) is regarded as the sequential importance sampling (SIS) procedure. However, the issue with SIS is that the importance weights will be concentrated on a few samples while the remainder of the samples become negligible after a few recursions. This is known as the degeneracy problem with a particle filter. A frequent approach to counter this problem is resampling the samples so that they are all equally weighted based on the posterior density. However, since resampling the samples introduces Monte Carlo error, resampling may not be performed in every recursion. It should only be executed when the distribution of the importance weight of the sample has been degraded. The state of the samples is determined by the effective sample size, which is defined by,

$$N_{eff} = \frac{N}{1 + var(w_k^*(i))}, \quad i = 1:N \qquad (33)$$

where $w_k^*(i)$ is the true weight of the sample,

$$w_k^*(i) = \frac{p(x_k|z_{1:k})}{q(x_k(i)|x_{k-1}(i))}, \quad i = 1:N \qquad (34)$$

However, as the true weight of the sample cannot be determined directly, the following method is used to approximate the effective sample size empirically with the normalized weights.

$$N_{eff} = \frac{1}{\sum_i^N w_i^2}, \quad i = 1:N \qquad (35)$$

Resampling is performed when $N_{eff}$ drops below a predetermined threshold $N_{th}$, which is done by relocating the samples with small weight to the samples with higher weights, hence, redistributing the weights of the particles.

One of the challenges of using an inertial navigation system is that it is sensitive to ferromagnetic and martensitic materials (e.g. Carbon steel), as well as permanent magnets (collectively, "magnetic materials"), which are commonly used materials in surgical instrumentation, as well as high power equipment. As part of the present system, the inertial system component uses a minimum of three magnetometers for detecting anomalies in the magnetic field. These magnetometers are placed in different locations in the unit. The outputs of the magnetometers change differently as an object composed of magnetic materials move into the vicinity of the unit. A detection algorithm is implemented to detect subtle changes among each magnetometer's output. Once calibrated, it is expected that the instantaneous magnitude of absolute difference of any two signal vectors, $M_1$, $M_2$, $M_3$, signals is near zero and each has instantaneous magnitude of approximately one.

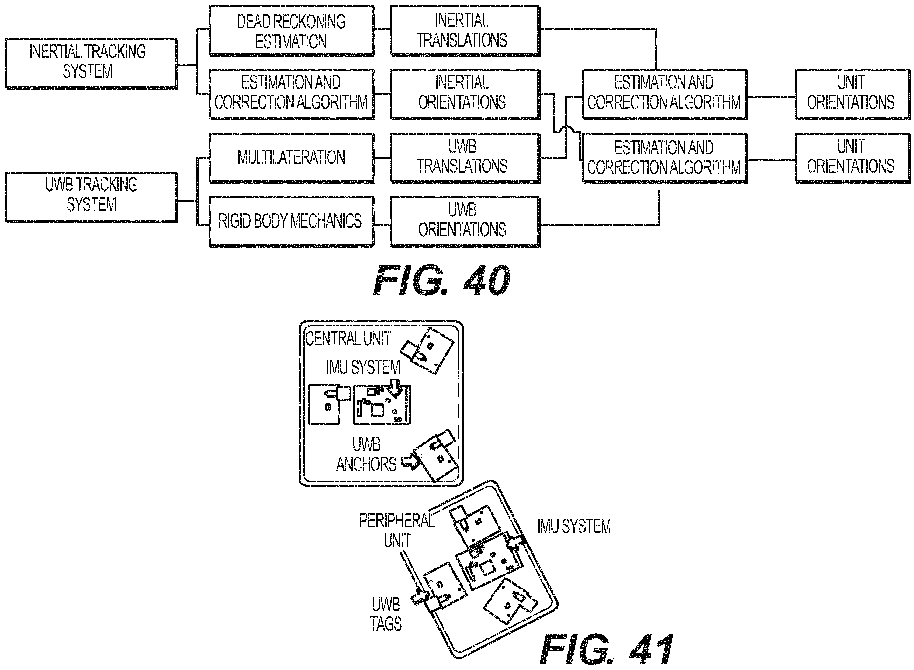

Referencing FIG. 40, a block diagram of determining the unit's translation and orientations is depicted. The exemplary hybrid inertial navigation and UWB system utilizes the advantages of each of the subsystems (i.e., IMU, UWB) to achieve subcentimeter accuracy in translation and subdegree in orientation. Estimation and correction algorithms (e.g., Kalman filter or particle filter) can be used to determine translations and orientations of the system. The linear acceleration from the inertial navigation system provides good estimates as to the translations of the system, while the UWB localization system provides a correction to transform the estimates into accurate translation data. For orientation, the inertial tracking system is sufficient to provide accurate orientations during normal operation. The orientation data from the UWB system is used primary for sanity checks and provide boundary conditions of the UWB navigation algorithm. However, upon detecting a magnetic anomaly from the inertial system, the magnetic sensors data is temporary disabled from the inertial data fusion algorithm. The heading orientation is tracked only based on the gyroscopes estimation.

The estimation of the heading orientation is subsequently corrected based on the UWB orientations calculation.

A proof of concept experiment was conducted to examine the orientation tracking of the UWB system with rigid body mechanics. FIG. 41 depicts the experimental setup. Two units were used during the experiment. For the central unit, three off-the-shelf UWB anchors and an IMU system were rigidly fixed together as a reference. For the peripheral unit, three off-the-shelf UWB tags and an IMU system were rigidly fixed together as an active navigation unit. In the first experiment, the initial orientation between the UWB and IMU systems was registered together as the initial orientation. The peripheral unit was rotated relative to the central unit and the orientations of each system were calculated. In the second experiment, both of the units were stationary. After the initial orientations of the units were registered, a ferromagnetic object was placed adjacent to the peripheral unit's IMU system to simulate a magnetic distortion situation.

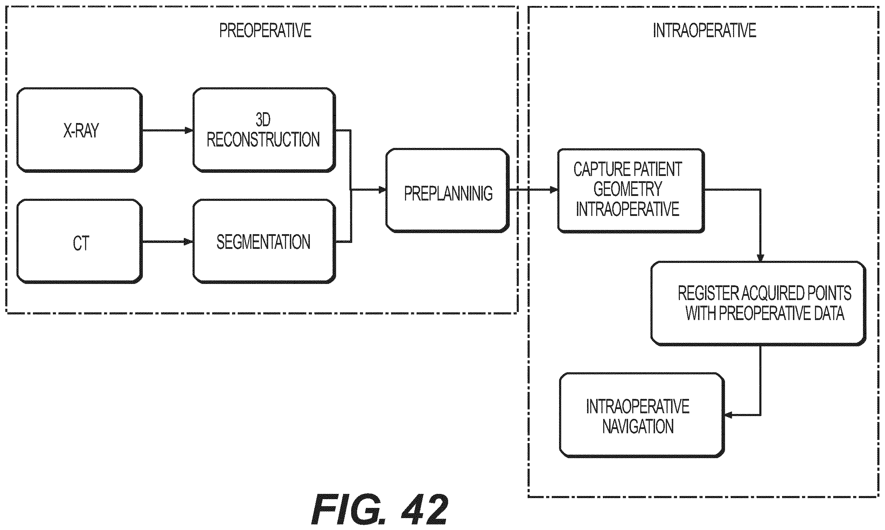

Turning to FIG. 42, when used as a surgical navigation system, the exemplary hybrid system can provide full navigation capability to the surgeon. The following outlines an exemplary application of the exemplary hybrid system for use with a total hip arthroplasty surgery. Preoperatively, the hip joint is imaged by an imaging modality. The output from the imaging modality is used to create patient specific anatomical virtual models. These models may be created using X-ray three dimensional reconstruction, segmentation of CT scans or MRI scans, or any other imaging modality from which a three dimensional virtual model can be created. Regardless of the approach taken to reach the patient specific model, the models are used for planning and placing both the acetabular component and femoral stem. The surgical planning data along with patient acetabulum and femoral anatomy are imported into the exemplary hybrid system.

For the femoral registration, in one exemplary configuration of this hybrid system, a central unit is attached to a patient's femur as a reference. A peripheral unit is attached to a mapping probe. In another exemplary configuration of this hybrid system, a central unit is positioned adjacent to an operating table as a global reference. A first peripheral unit is attached to a patient's femur, and a second peripheral unit is attached to a mapping probe. Using either configuration, the patient's exposed femoral anatomical surface is mapped by painting the surface with the probe. The collected surface points are registered with patient preoperative anatomical models. This translates the preoperative femoral planning into the operating room and registers it with the position of the patient's femur.

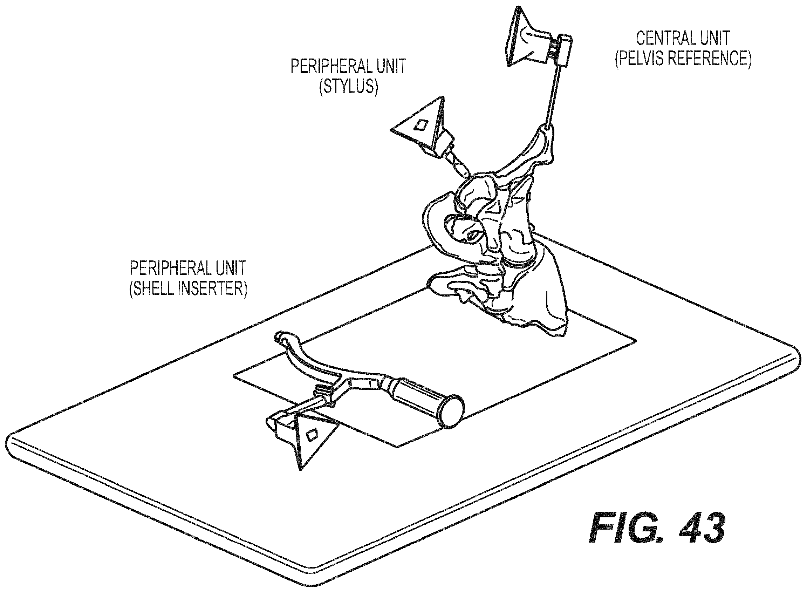

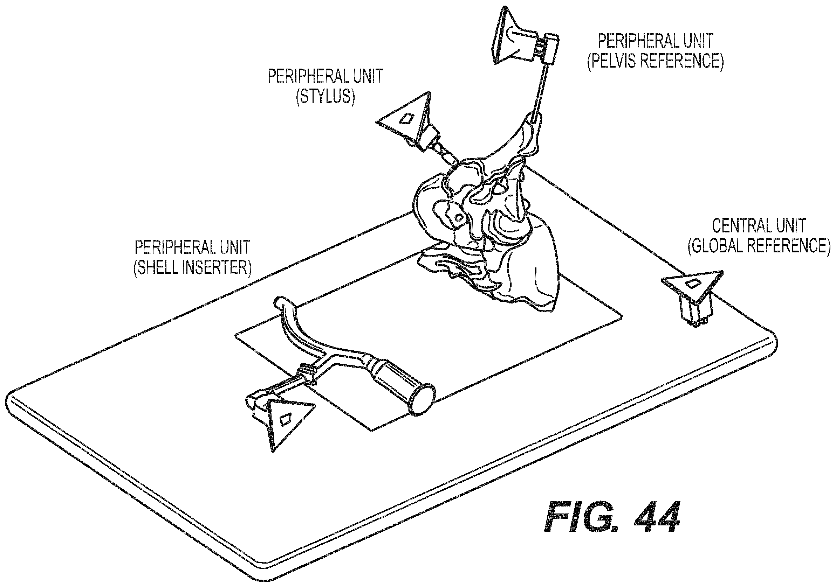

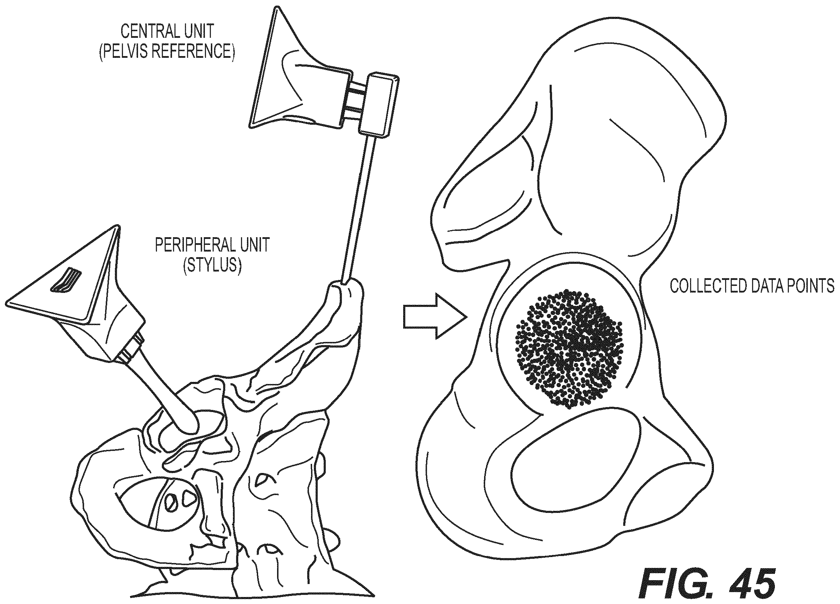

The registration of the patient's pelvis may take place after registration of the patient's femur. In one exemplary configuration of this hybrid system, a central unit is attached to the iliac crest of a patient's pelvis as a reference. A peripheral unit is attached to a mapping probe (see FIG. 43). In another exemplary configuration of this hybrid system, a central unit is positioned adjacent to the operating table. A first peripheral unit is attached to a patient's pelvis, and a second peripheral unit is attached to a mapping probe (see FIG. 44). Using either configuration, the patient's acetabular cup geometry is mapped by painting the surface with the probe. The collected surface points are registered with patient preoperative anatomical models (see FIG. 45). This translates the preoperative cup planning into the operating room and registers it with the position of the patient's pelvis.

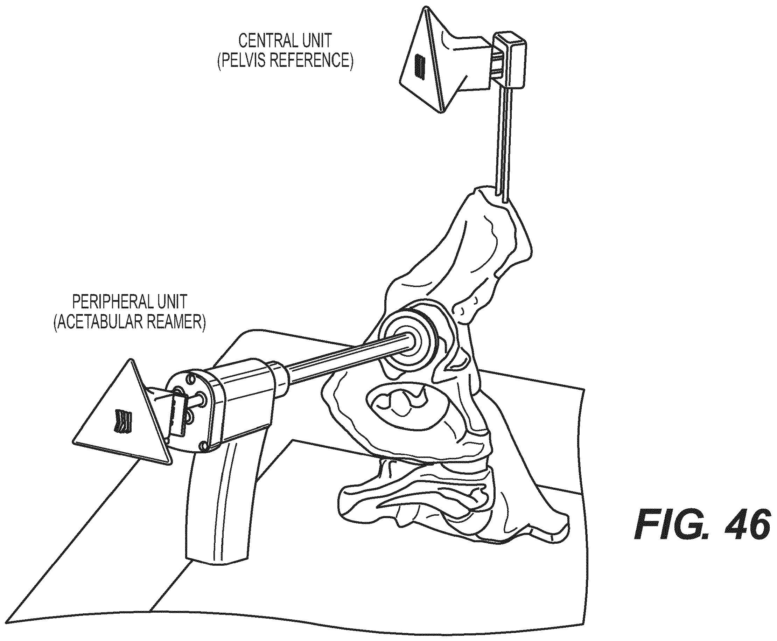

During the acetabular cup preparation, in one configuration of this hybrid system, a central unit is attached to the iliac crest of a patient's pelvis as a reference. A peripheral unit is attached to an acetabular reamer (see FIG. 46). In another alternate exemplary configuration of this invention, a central unit is positioned adjacent to the operating table. A first peripheral unit is attached to the iliac crest of a patient's pelvis, and a second peripheral unit is attached to an acetabular reamer. Using either configuration, the reaming direction is calculated by the differences between the relative orientations between the central and peripheral units, and the planned acetabular cup orientations having been predetermined as part of the preoperative surgical plan. In order to minimize error (e.g., deviation from the surgical plan), the surgeon may maneuver the acetabular reamer based on feedback from the surgical navigation guidance software indicating whether the position and orientation of the reamer coincide with the preoperative surgical plan. The reaming direction guidance may be provided to the surgeon via various viewing options such as 3D view, a clinical view, and multiple rendering options such as a computer rendering, an X-ray simulation, and a fluoroscopic simulation. The reaming depth is calculated by translational distances between the central and peripheral units. The surgeon uses this information to determine the reaming distance to avoid under or over reaming.

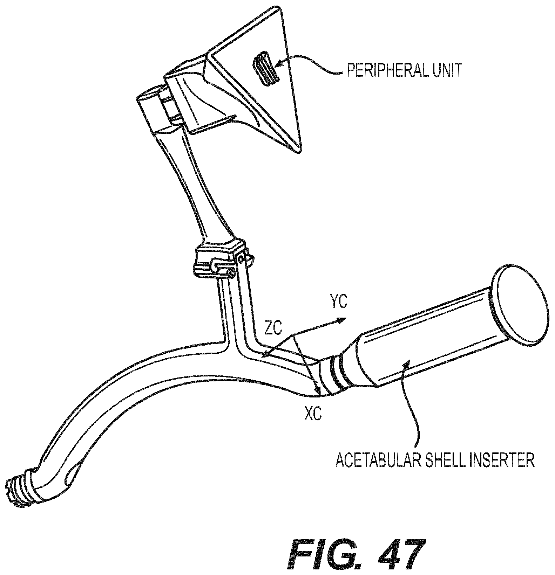

During the acetabular cup placement, in one configuration of this hybrid system, a central unit is attached to the iliac crest of a patient's pelvis as a reference. A peripheral unit is attached to an acetabular shell inserter (see FIG. 47). In another alternate exemplary configuration of this invention, a central unit is positioned adjacent to the operating table. A first peripheral unit is attached to the iliac crest of a patient's pelvis, and a second peripheral unit is attached to an acetabular shell inserter. Using either configuration, the reaming direction is calculated by the hybrid system using the differences between the relative orientations between the central and peripheral units, and the planned acetabular cup orientations predetermined via the preoperative surgical plan. In order to minimize error (e.g., deviation from the surgical plan), the surgeon may maneuver the acetabular inserter based on the surgical navigation guidance software of the hybrid system. The direction of the acetabular cup placement may be provided to the surgeon via various viewing options such as 3D view, a clinical view, and multiple rendering options such as a computer rendering, an X-ray simulation, and a fluoroscopic simulation. The acetabular cup placement depth is calculated by translational distances between the central and peripheral units. The surgeon uses this information to determine the final acetabular cup placement.

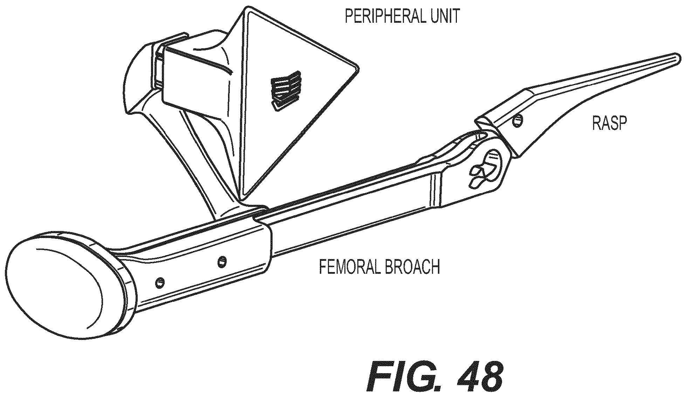

During the femoral stem preparation, in one exemplary configuration of this hybrid system, a central unit is attached to a patient's femur as a reference. A peripheral unit is attached to a femoral broach handle (see FIG. 48). In another alternate exemplary configuration of this invention, a central unit is positioned adjacent to the operating table. A first peripheral unit is attached to a patient's femur, and a second peripheral unit is attached to a femoral broach handle. Using either configuration, the broaching direction is calculated by the hybrid system using the differences between the relative orientations between the central and peripheral units, and the planned femoral stem orientations predetermined via the preoperative surgical plan. In order to minimize error (e.g., deviation from the surgical plan), the surgeon may maneuver the femoral broach based on the surgical navigation guidance software of the hybrid system. The broaching direction guidance is provided to the surgeon via various viewing options such as 3D view, a clinical view, and multiple rendering options such as a computer rendering, an X-ray simulation, and a fluoroscopic simulation. The broaching depth is calculated by translational distances between the central and peripheral units. The surgeon uses this information to determine the broached distance to avoid under or over rasping. In addition, the navigation software calculates and provides the overall leg length and offset based on the placement of the acetabular cup and the femoral broached depth.

During the femoral stem placement, in one exemplary configuration of this hybrid system, a central unit is attached to a patient's femur as a reference. A peripheral unit is attached to a femoral stem inserter. In another alternate exemplary configuration of this invention, a central unit is positioned adjacent to the operating table. A first peripheral unit is attached to a patient's femur, and a second peripheral unit is attached to a femoral stem inserter. Using either configuration, the placement direction is calculated by hybrid system using the differences between the relative orientations between the central and peripheral units, and the planned femoral stem orientations predetermined via the preoperative surgical plan. In order to minimize error (e.g., deviation from the surgical plan), the surgeon may maneuver the femoral stem inserter based on the surgical navigation guidance software. The direction of the femoral stem placement guidance is provided to the surgeon via various viewing options such as 3D view, a clinical view, and multiple rendering options such as a computer rendering, an X-ray simulation, and a fluoroscopic simulation. The femoral placement depth is calculated by translational distances between the central and peripheral units. The surgeon uses this information to determine the final femoral stem placement. The navigation software calculates and provides the overall leg length and offset.

The foregoing exemplary application of using the hybrid system during a total hip arthroplasty procedure can be applied to any number of other surgical procedures including, without limitation, total knee arthroplasty, total ankle arthroplasty, total shoulder arthroplasty, spinal surgery, open chest procedures, and minimally invasive surgical procedures.

Following from the above description, it should be apparent to those of ordinary skill in the art that, while the methods and apparatuses herein described constitute exemplary embodiments of the present invention, the invention described herein is not limited to any precise embodiment and that changes may be made to such embodiments without departing from the scope of the invention as defined by the claims. Additionally, it is to be understood that the invention is defined by the claims and it is not intended that any limitations or elements describing the exemplary embodiments set forth herein are to be incorporated into the interpretation of any claim element unless such limitation or element is explicitly stated. Likewise, it is to be understood that it is not necessary to meet any or all of the identified advantages or objects of the invention disclosed herein in order to fall within the scope of any claims, since the invention is defined by the claims and since inherent and/or unforeseen advantages of the present invention may exist even though they may not have been explicitly discussed herein.

**Claims**

1. A connected healthcare environment (100) comprising:

   a central repository (120) comprising a storage of patient information and data with patient-specific anatomical models, the central repository (120) additionally comprising cloud services (220), the central repository (120) communicatively coupled to at least one database comprising at least one of a statistical anatomical atlas (250) and a kinematic database (150), the central repository (120) configured to provide reports customized for the patient and the physician utilizing the patient information and data, the reports and central repository (120) being accessible by the patient by way of a patient portal (210), and by a physician by way of a physician portal (230);
   a computer running software defining the patient portal (210) comprising a software interface configured to generate instructions for displaying a virtual anatomical model of a patient's anatomy on a visual display, the software interface communicatively coupled to the central repository (120); and,
   a motion tracking device (270) communicatively coupled to the software interface and configured to transmit to the patient portal (210) the motion tracking data of the patient's anatomy as the anatomy is repositioned;
   wherein the software is configured to process the motion tracking data and generate instructions for dynamically displaying the virtual anatomical model in real time that mimics changing positions of the patient anatomy;
   wherein the patient portal (210) includes the software interface configured to collect the motion tracking data from the motion tracking device and to interface with the central repository (120); and
   wherein at least one of the customized reports accessible by way of the physician portal (230) utilizes the motion tracking data,
   wherein the central repository (120) is configured to store patient medical records,
   wherein the patient portal (210) is configured to collect orientation and position data and interface with the cloud services (220) to analyze the data, create reports for the patient that provide indicators of recovery progress, and update the patient medical records with the new information.

2. The connected healthcare environment of claim 1, wherein the statistical anatomical atlas (250) includes mathematical descriptions of at least one of bone, soft tissue, and connective tissue.

3. The connected healthcare environment of claim 2, wherein the mathematical descriptions are of bone, and the mathematical descriptions describe bones of an anatomical joint.

4. The connected healthcare environment of claim 2, wherein the mathematical descriptions are of bone, and the mathematical descriptions describe at least one of normal and abnormal bones.

5. The connected healthcare environment of claim 1, wherein the kinematic database (150) includes motion data associated with at least one of normal and abnormal kinematics.

6. The connected healthcare environment of claim 5, wherein the kinematic database (150) includes motion data associated with abnormal kinematics, and the motion data associated with abnormal kinematics includes a diagnosis for the abnormal kinematics.

7. The connected healthcare environment of any one of claims 1-6, wherein the motion tracking device (270) includes an inertial measurement unit.

8. The connected healthcare environment of any one of claims 1-7, wherein the motion tracking device (270) includes a plurality of inertial measurement units.

9. The connected healthcare environment of either claim 7 or 8, wherein the motion tracking device (270) includes ultrawide band electronics.

10. The connected healthcare environment of any of the foregoing claims, wherein the central repository (120) is communicatively coupled to the software interface.

11. The connected healthcare environment of any of the foregoing claims, further comprising a data acquisition station remote from, but communicatively coupled to, the central repository (120), the data acquisition station configured to access the stored patient information and data.

12. The connected healthcare environment of any one of the foregoing claims, further comprising a machine learning data structure (260) communicatively coupled to the central repository (120), the machine learning data structure (260) configured to generate a diagnosis using the motion tracking data.

13. The connected healthcare environment according to any preceding claim, wherein the central repository (120) comprises a local or cloud based storage of patient information and data.

**Patentansprüche**

1. Verbundene Gesundheitsfürsorgeumgebung (100), umfassend:

ein Zentralarchiv (120), das einen Speicher für Patienteninformationen und -daten mit patientenspezifischen anatomischen Modellen umfasst, wobei das Zentralarchiv (120) zusätzlich Cloud-Dienste (220) umfasst, das Zentralarchiv (120) kommunikationsmäßig mit mindestens einer Datenbank gekoppelt ist, die mindestens eines von einem statistischen anatomischen Atlas (250) und einer Kinematikdatenbank (150) umfasst, das Zentralarchiv (120) so konfiguriert ist, dass es Berichte bereitstellt, die unter Verwendung der Patienteninformationen und -daten für den Patienten und den Arzt maßgeschneidert werden, wobei die Berichte und das Zentralarchiv (120) für den Patienten über ein Patientenportal (210) und für einen Arzt über ein Arztportal (230) zugänglich sind; eine Software, die auf dem Computer läuft, die das Patientenportal (210) definiert, das eine Softwareschnittstelle umfasst, die so konfiguriert ist, dass sie Anweisungen zum Anzeigen eines virtuellen anatomischen Modells der Anatomie eines Patienten auf einer visuellen Anzeige erzeugt, wobei die Softwareschnittstelle kommunikationsmäßig mit dem Zentralarchiv (120) gekoppelt ist; und eine Bewegungsverfolgungsvorrichtung (270), die kommunikationsmäßig mit der Softwareschnittstelle gekoppelt und so konfiguriert ist, dass sie die Bewegungsverfolgungsdaten der Anatomie des Patienten an das Patientenportal (210) überträgt, wenn die Anatomie neu positioniert wird; wobei die Software so konfiguriert ist, dass sie die Bewegungsverfolgungsdaten verarbeitet und Anweisungen zum dynamischen Anzeigen des virtuellen anatomischen Modells, das sich verändernde Positionen der Patientenanatomie nachbildet, in Echtzeit erzeugt; wobei das Patientenportal (210) die Softwareschnittstelle beinhaltet, die so konfiguriert ist, dass sie die Bewegungsverfolgungsdaten von der Bewegungsverfolgungsvorrichtung sammelt und eine Schnittstelle zum Zentralarchiv (120) bildet; und wobei mindestens einer der maßgeschneiderten Berichte, die über das Arztportal (230) zugänglich sind, die Bewegungsverfolgungsdaten verwendet, wobei das Zentralarchiv (120) so konfiguriert ist, dass es Patientenakten speichert, wobei das Patientenportal (210) so konfiguriert ist, dass es Ausrichtungs- und Positionsdaten sammelt und eine Schnittstelle zu den Cloud-Diensten (220) bildet, um die Daten zu analysieren, Berichte für den Patienten zu erstellen, die Indikatoren für den Genesungsfortschritt bereitstellen, und die Patientenakten mit den neuen Informationen zu aktualisieren.

2. Verbundene Gesundheitsfürsorgeumgebung nach Anspruch 1, wobei der statistische anatomische Atlas (250) mathematische Beschreibungen mindestens eines von Knochen, Weichgewebe und Bindegewebe beinhaltet.

3. Verbundene Gesundheitsfürsorgeumgebung nach Anspruch 2, wobei die mathematischen Beschreibungen von Knochen sind und die mathematischen Beschreibungen Knochen eines anatomischen Gelenks beschreiben.

4. Verbundene Gesundheitsfürsorgeumgebung nach Anspruch 2, wobei die mathematischen Beschreibungen von Knochen sind und die mathematischen Beschreibungen mindestens eines von normalen und abnormalen Knochen beschreiben.

5. Verbundene Gesundheitsfürsorgeumgebung nach Anspruch 1, wobei die Kinematikdatenbank (150) Bewegungsdaten beinhaltet, die mindestens einem von normaler und abnormaler Kinematik zugeordnet sind.

6. Verbundene Gesundheitsfürsorgeumgebung nach Anspruch 5, wobei die Kinematikdatenbank (150) Bewegungsdaten beinhaltet, die abnormaler Kinematik zugeordnet sind, und die Bewegungsdaten, die abnormaler Kinematik zugeordnet sind, eine Diagnose für die abnormale Kinematik beinhalten.

7. Verbundene Gesundheitsfürsorgeumgebung nach einem der Ansprüche 1-6, wobei die Bewegungsverfolgungsvorrichtung (270) eine Trägheitsmesseinheit beinhaltet.

8. Verbundene Gesundheitsfürsorgeumgebung nach einem der Ansprüche 1-7, wobei die Bewegungsverfolgungsvorrichtung (270) eine Vielzahl von Trägheitsmesseinheiten beinhaltet.

9. Verbundene Gesundheitsfürsorgeumgebung nach entweder Anspruch 7 oder 8, wobei die Bewegungsverfolgungsvorrichtung (270) Ultrabreitband-Elektronik beinhaltet.

10. Verbundene Gesundheitsfürsorgeumgebung nach einem der vorstehenden Ansprüche, wobei das Zentralarchiv (120) kommunikationsmäßig mit der Softwareschnittstelle gekoppelt ist.

11. Verbundene Gesundheitsfürsorgeumgebung nach einem der vorstehenden Ansprüche, weiter eine Datenerfassungsstation umfassend, die vom Zentralarchiv (120) entfernt, aber kommunikationsmäßig mit diesem gekoppelt ist, wobei die Datenerfassungsstation so konfiguriert ist, dass sie auf die gespeicherten Patienteninformationen und -daten zugreift.

12. Verbundene Gesundheitsfürsorgeumgebung nach einem der vorstehenden Ansprüche, weiter eine Datenstruktur für maschinelles Lernen (260) umfassend, die kommunikationsmäßig mit dem Zentralarchiv (120) gekoppelt ist, wobei die Datenstruktur für maschinelles Lernen (260) so konfiguriert ist, dass sie unter Verwendung der Bewegungsverfolgungsdaten eine Diagnose erzeugt.

13. Verbundene Gesundheitsfürsorgeumgebung nach einem vorstehenden Anspruch, wobei das Zentralarchiv (120) einen lokalen oder Cloud-basierten Speicher für Patienteninformationen und -daten umfasst.

**Revendications**

1. Environnement de soins de santé connecté (100) comprenant :

un référentiel central (120) comprenant un stockage d'informations et de données sur le patient avec des modèles anatomiques spécifiques au patient, le référentiel central (120) comprenant en outre des services cloud (220), le référentiel central (120) couplé en communication à au moins une base de données comprenant au moins un d'un atlas anatomique statistique (250) et d'une base de données cinématique (150), le référentiel central (120) étant configuré pour fournir des rapports personnalisés pour le patient et le médecin en utilisant les informations et les données du patient, les rapports et le référentiel central (120) étant accessibles par le patient au moyen d'un portail patient (210), et par un médecin au moyen d'un portail médecin (230) ;

un logiciel exécuté sur ordinateur définissant le portail patient (210) comprenant une interface logicielle configurée pour générer des instructions pour afficher un modèle anatomique virtuel de l'anatomie d'un patient sur un affichage visuel, l'interface logicielle étant couplée en communication au référentiel central (120) ; et,

un dispositif de suivi de mouvement (270) couplé en communication à l'interface logicielle et configuré pour transmettre au portail patient (210) les données de suivi de mouvement de l'anatomie du patient lorsque l'anatomie est repositionnée ;

dans lequel le logiciel est configuré pour traiter les données de suivi de mouvement et générer des instructions pour afficher dynamiquement le modèle anatomique virtuel en temps réel qui imite les positions changeantes de l'anatomie du patient ;

dans lequel le portail patient (210) inclut l'interface logicielle configurée pour collecter les données de suivi de mouvement provenant du dispositif de suivi de mouvement et pour s'interfacer avec le référentiel central (120) ; et dans lequel au moins un des rapports personnalisés accessibles via le portail médecin (230) utilise les données de suivi de mouvement,

dans lequel le référentiel central (120) est configuré pour stocker les dossiers médicaux des patients,

dans lequel le portail patient (210) est configuré pour collecter des données d'orientation et de position et s'interfacer avec les services cloud (220) pour analyser les données, créer des rapports pour le patient qui fournissent des indicateurs de progression de la récupération, et mettre à jour les dossiers médicaux du patient avec les nouvelles informations.

2. Environnement de soins de santé connecté selon la revendication 1, dans lequel l'atlas anatomique statistique (250) inclut des descriptions mathématiques d'au moins un élément parmi les os, les tissus mous et les tissus conjonctifs.

3. Environnement de soins de santé connecté selon la revendication 2, dans lequel les descriptions mathématiques concernent des os, et les descriptions mathématiques décrivent des os d'une articulation anatomique.

4. Environnement de soins de santé connecté selon la revendication 2, dans lequel les descriptions mathématiques concernent des os, et les descriptions mathématiques décrivent au moins un des os normaux ou anormaux.

5. Environnement de soins de santé connecté selon la revendication 1, dans lequel la base de données cinématique (150) inclut des données de mouvement associées à au moins une des cinématiques normales et anormales.

6. Environnement de soins de santé connecté selon la revendication 5, dans lequel la base de données cinématique (150) inclut des données de mouvement associées à la cinématique anormale, et les données de mouvement associées à la cinématique anormale incluent un diagnostic pour la cinématique anormale.

7. Environnement de soins de santé connecté selon l'une quelconque des revendications 1 à 6, dans lequel le dispositif de suivi de mouvement (270) inclut une unité de mesure inertielle.

8. Environnement de soins de santé connecté selon l'une quelconque des revendications 1 à 7, dans lequel le dispositif de suivi de mouvement (270) inclut une pluralité d'unités de mesure inertielle.

9. Environnement de soins de santé connecté selon la revendication 7 ou 8, dans lequel le dispositif de suivi de mouvement (270) inclut des composants électroniques à bande ultra-large.

10. Environnement de soins de santé connecté selon l'une quelconque des revendications précédentes, dans lequel le référentiel central (120) est couplé en communication à l'interface logicielle.

11. Environnement de soins de santé connecté selon l'une quelconque des revendications précédentes, comprenant en outre une station d'acquisition de données distante du référentiel central (120), mais couplée en communication avec celui-ci, la station d'acquisition de données étant configurée pour accéder aux informations et données stockées sur le patient.

12. Environnement de soins de santé connecté selon l'une quelconque des revendications précédentes, comprenant en outre une structure de données d'apprentissage automatique (260) couplée en communication au référentiel central (120), la structure de données d'apprentissage automatique (260) étant configurée pour générer un diagnostic à l'aide des données de suivi de mouvement.

13. Environnement de soins de santé connecté selon une quelconque revendication précédente, dans lequel le référentiel central (120) comprend un stockage local ou basé sur un cloud d'informations et de données sur le patient.

**FIG. 1**

EP 3 422 951 B1

FIG. 2

150 — KINEMATIC DATABASE

240 — ELECTRONIC MEDICAL RECORD

250 — STATISTICAL ANATOMICAL ATLASES

260 — MACHINE LEARNING DATA STRUCTURE

200

210 — PATIENT PORTAL
- GATHERING MOTION DATA
- REPORT PROGRESS
- GATHERING PROMS

180 — PATIENT

220 — CLOUD SERVICES
- RETRIEVE/UPDATE EMR
- RETRIEVE/UPDATE PATIENT DATA
- INCOMING DATA OPTIMIZATION
- ANALYZE DATA VIA DEEP LEARNING

230 — CLINICIAN PORTAL
- REPORT TREATMENT SUGGESTION
- REPORT OPTIMAL SURGICAL PLAN
- REPORT PATIENT PROGRESS
- REPORT READMISSION WARNING
- REPORT LONG TERM OUTCOMES & PERFORMANCE
- GATHERING MOTION DATA

170 — CLINICIAN

270 — INERTIAL/UWB MOTION TRACKING SENSORS

EP 3 422 951 B1

**FIG. 3**

**FIG. 4**

EP 3 422 951 B1

**FIG. 5**

**FIG. 6**

EP 3 422 951 B1

**CALIBRATION: BY HAND**  2:53 PM  99%  2:3

CALIBRATION STEP 02:

TURN THE POD ON ITS SIDE
SO THE LOGO IS FACING
RIGHT, THEN SLOWLY ROTATE
THE POD FORWARD ONE
FULL REVOLUTION.

| BACK | CONTINUE |

## FIG. 7

**CALIBRATION: BY HAND**  2:53 PM  99%  3:3

CALIBRATION STEP 03:

ROTATE THE POD A QUARTER
TURN COUNTER-CLOCKWISE
UNTIL THE LOGO IS BACK ON THE
TOP,THEN CONTINUE SLOWLY
ROTATING ONE FULL REVOLUTION
COUNTER-CLOCKWISE.

| BACK | FINALIZE |

## FIG. 8

FIG. 9

EP 3 422 951 B1

LOCAL MAGNETIC FIELD MAP
(RAW, ISOMETRIC VIEW)

**FIG. 10**

LOCAL MAGNETIC FIELD MAP
(CALIBRATED, ISOMETRIC VIEW)

LOCAL MAGNETIC FIELD MAP
(RAW, FRONT VIEW)

LOCAL MAGNETIC FIELD MAP
(CALIBRATED, FRONT VIEW)

*FIG. 10*
*CONT.*

LOCAL MAGNETIC FIELD MAP
(RAW, TOP VIEW)

LOCAL MAGNETIC FIELD MAP
(CALIBRATED, TOP VIEW)

## FIG. 10

### CONT.-1

EP 3 422 951 B1

FIG. 11A

FIG. 11B

FIG. 11C

38

**FIG. 12**

**FIG. 13**

●●○○○ 🛜     2:54 PM     ✳   99% 🔋

≡      ≋TECHMAH

← 

AVAILABLE EXERCISES
PLEASE CHOOSE AN EXERCISE TO ADVANCE

🖐 LEG EXTENSION

🖐 SHOULDER EXTENSION

CONTINUE

**FIG. 14**

●●○○○ 🛜     2:55 PM     ✳   99% 🔋

≡      ≋TECHMAH

←     PLACE PODS
BEFORE BEGINNING THE SCENE, PLEASE
ENSURE EACH POD IS POSITIONED TO MATCH
THE KEY BELOW

POD A = FEMUR

CONTINUE

**FIG. 15**

40

**FIG. 16**

3:35 PM ≯ 91%

☰ ≡TECHMAH

PLACE PODS
BEFORE BEGINNING THE SCENE, PLEASE
ENSURE EACH POD IS POSITIONED TO MATCH
THE KEY BELOW
POD A - TIBIA

BEFORE ADVANCING,
PLEASE ENSURE PODS
ARE SECURE AND

BEGIN

CONTINUE

**FIG. 17**

EP 3 422 951 B1

FIG. 19

FIG. 18

**FIG. 20**

**FIG. 21**

EP 3 422 951 B1

**FIG. 22**

FIG. 23

CURRENT QUATERNION1 - REFERENCE QUATERNION1

$[I_{3x3}]$ PROJECTION

IMU AXIS IN
SUPERIOR-INFERIOR DIRECTION

IMU AXIS IN
MEDIAL-LATERAL
DIRECTION

UNIT 1

IMU AXIS IN
ANTERIOR-POSTERIOR
DIRECTION

ABSOLUTE ANGLE
OF L1 RELATIVE TO L5
IN THE SUPERIOR-
INFERIOR DIRECTION

CURRENT QUATERNION2 - REFERENCE QUATERNION2

$[I_{3x3}]$ PROJECTION

IMU AXIS IN
SUPERIOR-INFERIOR DIRECTION

IMU AXIS IN
MEDIAL-LATERAL
DIRECTION

UNIT 2

IMU AXIS IN
ANTERIOR-POSTERIOR
DIRECTION

*FIG. 24*

FIG. 25B

FIG. 25A

FIG. 26B

FIG. 26A

FIG. 27

FIG. 28

FIG. 29A

FIG. 29B

FIG. 29C

FIG. 30A          FIG. 30B          FIG. 30C

EP 3 422 951 B1

FIG. 31

**FIG. 32A**

**FIG. 32B**

FIG. 33A

FIG. 33B

FIG. 33C

60°

20°

EP 3 422 951 B1

**FIG. 34**

UWB ANTENNA

UWB TRANSCEIVER

UWB ANTENNA

UWB ANTENNA

INERTIAL TRACKING SYSTEM

**FIG. 35**

ANECHOIC CHAMBER (TOP VIEW)

"APPARENT PHASE CENTER"

X

Y   Z

-45°

| UWB ANTENNA |

1.5m

| UWB ANTENNA |

+45°

0     39.5mm

| RX |

| TX |

# *FIG. 36*

| MICROCONTROLLER | | UWB TRANSCEIVER | | POWER AMPLIFER | | MULTIPLEXER |

| ANTENNA |

| ANTENNA |

| ANTENNA |

| ANTENNA |

# *FIG. 37*

**FIG. 38**

**FIG. 39**

```
┌─────────────────┐        ┌──────────────────┐    ┌──────────────────┐
│ INERTIAL TRACKING│───┬───▶│  DEAD RECKONING  │───▶│     INERTIAL     │──┐
│     SYSTEM       │   │    │    ESTIMATION    │    │   TRANSLATIONS   │  │
└─────────────────┘   │    └──────────────────┘    └──────────────────┘  │
                      │    ┌──────────────────┐    ┌──────────────────┐  │   ┌──────────────────┐   ┌──────────────┐
                      └───▶│  ESTIMATION AND  │───▶│     INERTIAL     │  ├──▶│  ESTIMATION AND  │──▶│     UNIT     │
                           │CORRECTION ALGORITHM│   │   ORIENTATIONS   │  │   │CORRECTION ALGORITHM│  │ ORIENTATIONS │
                           └──────────────────┘    └──────────────────┘  │   └──────────────────┘   └──────────────┘
                                                                          │
┌─────────────────┐        ┌──────────────────┐    ┌──────────────────┐  │   ┌──────────────────┐   ┌──────────────┐
│   UWB TRACKING  │───┬───▶│  MULTILATERATION │───▶│       UWB        │  ├──▶│  ESTIMATION AND  │──▶│     UNIT     │
│     SYSTEM      │   │    │                  │    │   TRANSLATIONS   │  │   │CORRECTION ALGORITHM│  │ ORIENTATIONS │
└─────────────────┘   │    └──────────────────┘    └──────────────────┘  │   └──────────────────┘   └──────────────┘
                      │    ┌──────────────────┐    ┌──────────────────┐
                      └───▶│RIGID BODY MECHANICS│──▶│       UWB        │
                           │                  │    │   ORIENTATIONS   │
                           └──────────────────┘    └──────────────────┘
```

## FIG. 40

CENTRAL UNIT

IMU SYSTEM

UWB
ANCHORS

PERIPHERAL
UNIT

IMU SYSTEM

UWB
TAGS

## FIG. 41

**FIG. 42**

CENTRAL UNIT
(PELVIS REFERENCE)

PERIPHERAL UNIT
(STYLUS)

PERIPHERAL UNIT
(SHELL INSERTER)

**FIG. 43**

PERIPHERAL UNIT
(PELVIS REFERENCE)

PERIPHERAL UNIT
(STYLUS)

CENTRAL UNIT
(GLOBAL REFERENCE)

PERIPHERAL UNIT
(SHELL INSERTER)

FIG. 44

EP 3 422 951 B1

CENTRAL UNIT
(PELVIS REFERENCE)

PERIPHERAL UNIT
(STYLUS)

COLLECTED DATA POINTS

FIG. 45

CENTRAL UNIT
(PELVIS REFERENCE)

PERIPHERAL UNIT
(ACETABULAR REAMER)

FIG. 46

PERIPHERAL UNIT

YC

ZC

XC

ACETABULAR SHELL INSERTER

**FIG. 47**

EP 3 422 951 B1

PERIPHERAL UNIT

RASP

FEMORAL BROACH

FIG. 48

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016007936 A
- WO 2015089118 A
- WO 2015054290 A